# EUROPEAN PATENT SPECIFICATION

(11) **EP 3 847 195 B1**
(45) Date of publication and mention of the grant of the patent: **05.03.2025**
(21) Application number: 19854895.0
(22) Date of filing: 29.08.2019
(51) Int. Cl.: C07K 16/28, C07K 16/30, A61K 39/395, A61P 35/00, C07K 16/40

(54) **CHIMERIC ANTIGEN RECEPTOR CELLS FOR TREATING SOLID TUMOR**
CHIMÄRE ANTIGEN-REZEPTORZELLEN ZUR BEHANDLUNG VON SOLIDEN TUMOREN
CELLULES DE RÉCEPTEUR D'ANTIGÈNE CHIMÉRIQUE POUR LE TRAITEMENT D'UNE TUMEUR SOLIDE

(30) Priority: 30.08.2018 US 201862725025 P; 13.09.2018 US 201862731079 P; 01.11.2018 US 201862754175 P; 19.02.2019 US 201962807482 P; 03.05.2019 US 201962842936 P; 16.05.2019 US 201962848984 P
(43) Date of publication of application: 14.07.2021
(73) Proprietor: Innovative Cellular Therapeutics Holdings, Ltd., Grand Cayman, KYI - 1104 (KY); Innovative Cellular Therapeutics Inc., Rockville, MD 20850 (US)
(72) Inventor: XIAO, Lei, Rockville, MD 20850 (US); SUN, He, Shanghai, 201203 (CN); CAO, Zhiyuan, Shanghai, 201203 (CN); WANG, Wensheng, Shanghai, 201203 (CN); PU, Chengfei, Shanghai, 201203 (CN); MAO, Li, Shanghai, 201203 (CN)
(74) Representative: Potter Clarkson
(86) International application number: PCT/US2019/048890
(87) International publication number: WO 2020/047306

(56) References cited:
- WO-A1-2018/023976
- WO-A1-2018/023976
- WO-A1-2020/106843
- WO-A1-2020/146743
- WO-A1-83/03679
- NO-B- 168 969
- US-A1- 2013 108 609
- US-A1- 2016 362 472
- US-A1- 2016 362 472
- ANONYMOUS: "Anti-ACPP Product Datasheet", December 2012 (2012-12-01), pages 1 - 1, XP055695448, Retrieved from the Internet <URL:https://www.atlasantibodies.com/api/print_datasheet/HPA004335.pdf> [retrieved on 20191031]

## Description

### TECHNICAL FIELD

The present disclosure relates to modified cells and uses, in particular to compositions and methods for treating cancer using Chimeric Antigen Receptor (CAR) cells.

### BACKGROUND

Most existing cancer treatment programs include surgery, radiotherapy, and chemotherapy, targeted therapy and immunotherapy. The drawbacks of the existing programs include poor treatment of advanced patients, side effects, patients with poor quality of life. For example, treatment of renal cancer includes resection, targeted therapy (anti-VEGF and mTOR inhibitor, etc.) and immunotherapy (IL-2, PD1 antibody, etc.). Treatment of pancreatic cancer includes surgical resection, radiotherapy, and chemotherapy. Treatment of urothelial carcinoma includes surgical resection, chemoradiation, targeted therapy, and immunotherapy. Treatment of breast cancer includes surgical resection, chemoradiation, targeted therapy, and immunotherapy. Treatment of ovarian cancer includes surgical resection, radiotherapy, chemotherapy, and targeted therapy. Treatment of prostate cancer includes surgical resection, chemoradiation, targeted therapy, and Immunotherapy. Treatment of esophageal cancer includes surgical resection, radiotherapy, and chemotherapy. Treatment of colorectal cancer includes surgical resection, radiotherapy, chemotherapy, and targeted therapy. Treatment of endometrial cancer: surgical resection, radiotherapy, chemotherapy, and targeted therapy.
WO 83/03679 describes polydomas that produce a hybrid monoclonal antibody having a dual specificity against two different antigenic determinants.
US 2013/108609 describes a transmembrane prostatic acid phosphatase (TM-PAP) protein or the C-terminal part thereof.
NO 168 969 describes a monoclonal antibody again PAP.

### SUMMARY

Embodiments relate to a discovery that some antigens have relatively low expression on tumor cells, as compared to the expression on normal tissues. Further, while expressed in normal tissues, some other antigens are specifically expressed in a certain tissue (e.g., a group of cells or an organ), and the killing of normal cells of the tissue does not cause a life-threatening event (e.g., complications) to the subject. Examples of the nonessential tissues include organs such as prostate, breast, or melanocyte. Accordingly, the embodiments of the present disclosure relate to a chimeric antigen receptor (CAR) including an extracellular domain that binds at least one of these antigens and treating the cancer using cells including the CAR.

Embodiments relate to compositions and methods for treating cancer using CAR cells. Embodiments relate to an isolated nucleic acid sequence encoding a CAR, wherein the CAR comprises an extracellular domain, a transmembrane domain, and an intracellular domain, wherein the extracellular domain of the CAR binds an antigen of a solid tumor. In some disclosures the antigen may comprise SIGLEC15, SLC6A3, KISS1R, QRFPR, GPR119, CLDN6, UPK2, ADAM12, SLC45A3, ACPP, MUC21, MUC16, MS4A12, ALPP, SLC2A14, GS1-259H13.2, ERVFRD-1, ADGRG2, ECEL1, CHRNA2, GP2, or PSG9.

### BRIEF DESCRIPTION OF THE DRAWINGS

The Detailed Description is described with reference to the accompanying figures. The use of the same reference numbers in different figures indicates similar or identical items.

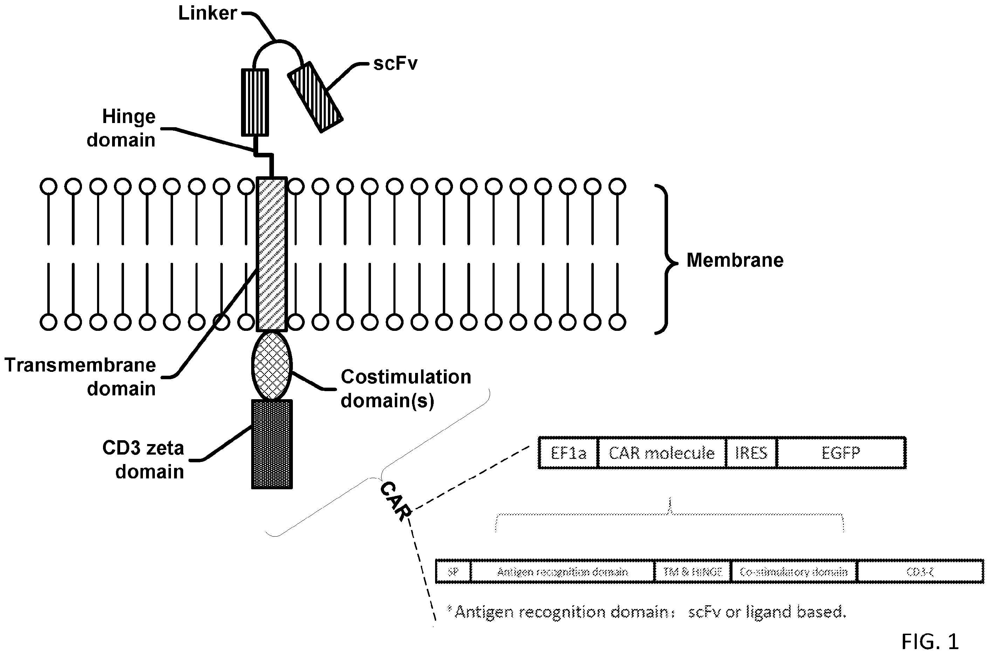

FIG. 1 shows a schematic diagram illustrating an example of a CAR structure.
FIG. 2 shows flow cytometry analysis of T cells expressing CARs.
FIG. 3 shows results of a killing assay of anti-SIGLEC15 CAR.
FIGS. 4 and 5 show results of cytokine release assays of anti-SIGLEC15 CAR.
FIG. 6 shows results of a killing assay of KISSR CAR.
FIG. 7 show results of a cytokine release assay of anti-KISSR CAR.
FIGs. 8, 9, and 10 show flow cytometry assay for ACPP antibodies.
FIG. 11 shows immunofluorescence staining of paraffin sections of prostate cancer.
FIG. 12 shows 293T cells expressing ACPP.
FIG. 13 shows cytometry assay of T cells expressing ACPP CAR.
FIG. 14 shows results of co-culturing assay.
FIG. 15 shows IFN-γ release of anti-ACPP CAR T cells in response to ACPP.
FIG. 16 shows that CAR expression of anti-ACPP CAR on T cells can be measured by detecting human CAR antibody.
FIG. 17 shows cytokine release assay of anti-ACPP CAR T cells.
FIGS. 18 and 19 show CD137 expression in various conditions.
FIG. 20 shows antibody titer against UPK2-His in mouse serum after ELISA assay.
FIG. 21 shows analysis of binding of 8 phage display antibodies to UPK2-His based on Phage-ELISA.
FIG. 22 shows ELISA analysis of the binding of recombinant monoclonal antibodies to recombinant UPK2-His.
FIG. 23 shows ELISA analysis of the specificity of recombinant anti-UPK2 mAb.

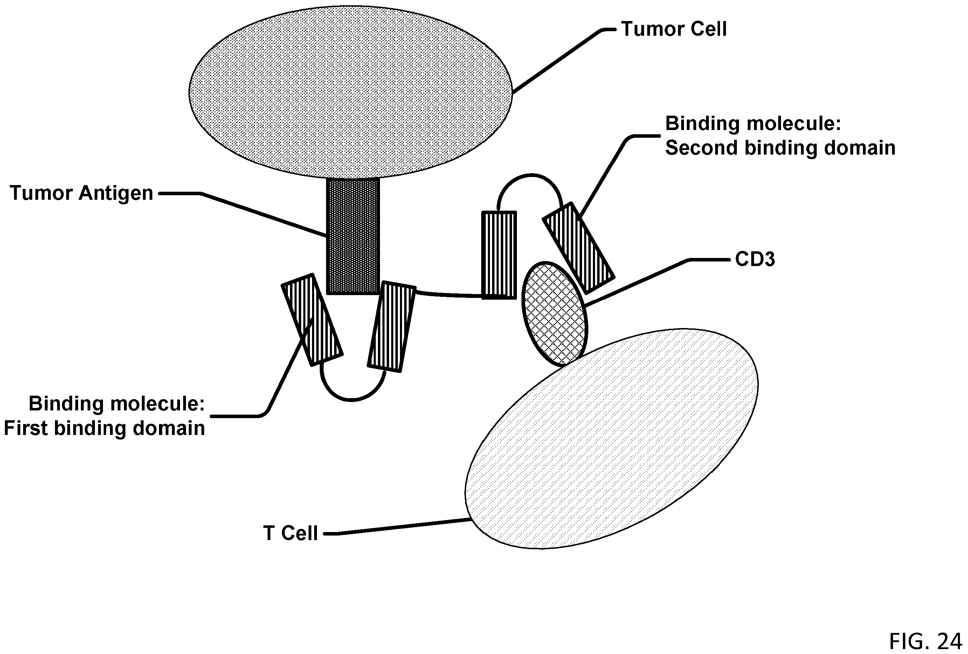

FIG. 24 shows an exemplary structure of a binding molecule.

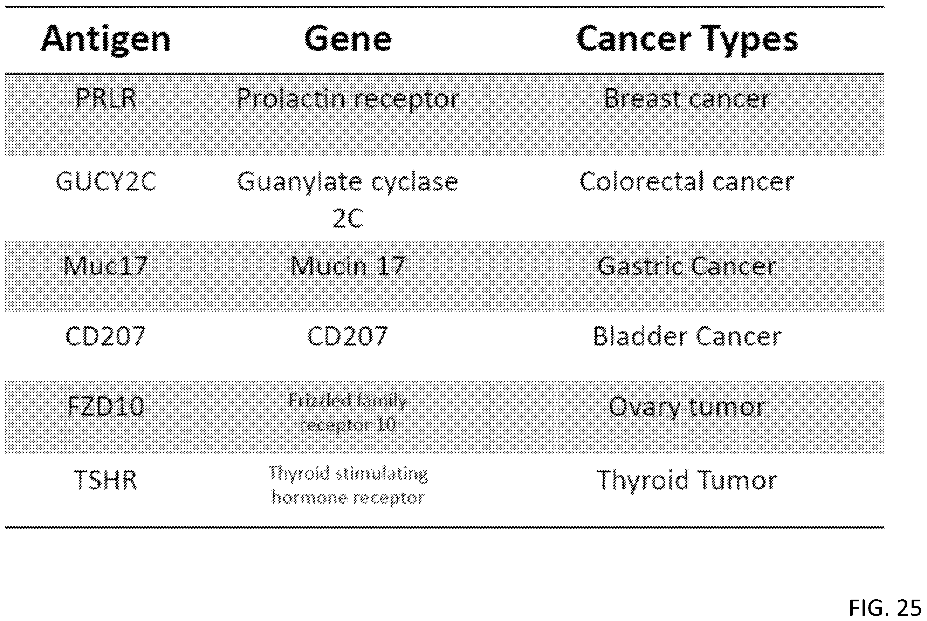

FIG. 25 shows a table of markers and the uses thereof.
FIG. 26 shows an exemplary structure of a CAR.

### DETAILED DESCRIPTION

The invention is set out in the claims and provides an antibody that binds prostatic acid phosphatase (ACPP), wherein the antibody comprises:
a heavy chain variable region (HVR) comprising amino acid sequence SEQ ID NO: 83 and a light chain variable region (LVR) comprising amino acid sequence SEQ ID NO: 82. The invention is defined by the claims and any other aspects, configurations or embodiments set forth herein not falling within the scope of the claims are for information only. Any references in the description to methods of treatment refer to the compounds, pharmaceutical compositions and medicaments of the present invention for use in a method for treatment of the human (or animal) body by therapy or for diagnosis.

The invention also provides a chimeric antigen receptor (CAR) comprising an antigen binding domain comprising an antibody that comprises a heavy chain variable region (HVR) comprising amino acid sequence SEQ ID NO: 83 and a light chain variable region (LVR) comprising amino acid sequence SEQ ID NO: 82.

The invention also provides a polynucleotide that encodes the antibody or CAR of the invention, and also provides a modified cell that comprises said polynucleotide.

Unless defined otherwise, all technical and scientific terms used herein have the same meaning as commonly understood by those of ordinary skill in the art to which the disclosure belongs. Although any method and material similar or equivalent to those described herein can be used in the practice or testing of the present disclosure, preferred methods and materials are described. For the purposes of the present disclosure, the following terms are defined below.

The articles "a" and "an" are used herein to refer to one or to more than one (i.e., to at least one) of the grammatical object of the article. By way of example, "an element" means one element or more than one element.

By "about" is meant a quantity, level, value, number, frequency, percentage, dimension, size, amount, weight or length that varies by as much as 20, 15, 10, 9, 8, 7, 6, 5, 4, 3, 2 or 1% to a reference quantity, level, value, number, frequency, percentage, dimension, size, amount, weight or length.

The term "activation," as used herein, refers to the state of a cell that has been sufficiently stimulated to induce detectable cellular proliferation. Activation can also be associated with induced cytokine production and detectable effector functions. The term "activated T cells" refers to, among other things, T cells that are undergoing cell division.

The term "antibody" is used in the broadest sense and refers to monoclonal antibodies (including full length monoclonal antibodies), polyclonal antibodies, multi-specific antibodies (e.g., bispecific antibodies), and antibody fragments so long as they exhibit the desired biological activity or function. The antibodies in the present disclosure may exist in a variety of forms including, for example, polyclonal antibodies, monoclonal antibodies, Fv, Fab and F(ab)₂, as well as single chain antibodies and humanized antibodies (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426).

The term "antibody fragments" refers to a portion of a full length antibody, for example, the antigen binding or variable region of the antibody. Other examples of antibody fragments include Fab, Fab', F(ab')2, and Fv fragments; diabodies; linear antibodies; single-chain antibody molecules; and multi-specific antibodies formed from antibody fragments.

The term "Fv" refers to the minimum antibody fragment which contains a complete antigen-recognition and -binding site. This fragment consists of a dimer of one heavy- and one light-chain variable region domain in tight, non-covalent association. From the folding of these two domains emanates six hypervariable loops (3 loops each from the H and L chain) that contribute the amino acid residues for antigen binding and confer antigen binding specificity to the antibody. However, even a single variable domain (or half of a Fv including only three complementarity determining regions (CDRs) specific for an antigen) has the ability to recognize and bind antigen, although at a lower affinity than the entire binding site (the dimer).

An "antibody heavy chain," as used herein, refers to the larger of the two types of polypeptide chains present in all antibody molecules in their naturally occurring conformations. An "antibody light chain," as used herein, refers to the smaller of the two types of polypeptide chains present in all antibody molecules in their naturally occurring conformations. κ and λ light chains refer to the two major antibody light chain isotypes.

The term "synthetic antibody" refers to an antibody which is generated using recombinant DNA technology, such as, for example, an antibody expressed by a bacteriophage. The term also includes an antibody which has been generated by the synthesis of a DNA molecule encoding the antibody and the expression of the DNA molecule to obtain the antibody, or to obtain an amino acid encoding the antibody. The synthetic DNA is obtained using technology that is available and well known in the art.

The term "antigen" refers to a molecule that provokes an immune response, which may involve either antibody production, or the activation of specific immunologically-competent cells, or both. Antigens include any macromolecule, including all proteins or peptides, or molecules derived from recombinant or genomic DNA. For example, DNA including a nucleotide sequence or a partial nucleotide sequence encoding a protein or peptide that elicits an immune response, and therefore, encodes an "antigen" as the term is used herein. An antigen need not be encoded solely by a full-length nucleotide sequence of a gene. An antigen can be generated, synthesized or derived from a biological sample including a tissue sample, a tumor sample, a cell, or a biological fluid.

The term "anti-tumor effect" as used herein, refers to a biological effect associated with a decrease in tumor volume, a decrease in the number of tumor cells, a decrease in the number of metastases, decrease in tumor cell proliferation, decrease in tumor cell survival, an increase in life expectancy of a subject having tumor cells, or amelioration of various physiological symptoms associated with the cancerous condition. An "anti-tumor effect" can also be manifested by the ability of the peptides, polynucleotides, cells, and antibodies in the prevention of the occurrence of tumor in the first place.

The term "auto-antigen" refers to an antigen mistakenly recognized by the immune system as being foreign. Auto-antigens include cellular proteins, phosphoproteins, cellular surface proteins, cellular lipids, nucleic acids, glycoproteins, including cell surface receptors.

The term "autologous" is used to describe a material derived from a subject which is subsequently re-introduced into the same subject.

The term "allogeneic" is used to describe a graft derived from a different subject of the same species. As an example, a donor subject may be a related or unrelated or recipient subject, but the donor subject has immune system markers which are similar to the recipient subject.

The term "xenogeneic" is used to describe a graft derived from a subject of a different species. As an example, the donor subject is from a different species than a recipient subject and the donor subject and the recipient subject can be genetically and immunologically incompatible.

The term "cancer" refers to a disease characterized by the rapid and uncontrolled growth of aberrant cells. Cancer cells can spread locally or through the bloodstream and lymphatic system to other parts of the body. Examples of various cancers include breast cancer, prostate cancer, ovarian cancer, cervical cancer, skin cancer, pancreatic cancer, colorectal cancer, renal cancer, liver cancer, brain cancer, lymphoma, leukemia, lung cancer, and the like.

Cancers that may be treated include tumors that are not vascularized, or not yet substantially vascularized, as well as vascularized tumors. The cancers may include non-solid tumors (such as hematological tumors, for example, leukemias and lymphomas) or may include solid tumors. Types of cancers to be treated with the CARs of the disclosure include, but are not limited to, carcinoma, blastoma, and sarcoma, and certain leukemia or lymphoid malignancies, benign and malignant tumors, and malignancies, e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors, such as sarcomas and carcinomas, include fibrosarcoma, myxosarcoma, liposarcoma, chondrosarcoma, osteosarcoma, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, medullary thyroid carcinoma, papillary thyroid carcinoma, pheochromocytomas sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, seminoma, bladder carcinoma, melanoma, and CNS tumors (such as a glioma (such as brainstem glioma and mixed gliomas), glioblastoma (also known as glioblastoma multiforme), astrocytoma, CNS lymphoma, germinoma, medulloblastoma, Schwannoma craniopharyngioma, ependymoma, pinealoma, hemangioblastoma, acoustic neuroma, oligodendroglioma, meningioma, neuroblastoma, retinoblastoma, and brain metastases).

A solid tumor antigen is an antigen expressed on a solid tumor. In embodiments, solid tumor antigens are also expressed at low levels on healthy tissue. Examples of solid tumor antigens and their related disease tumors are provided in Table 1.

**Table 1**

| **Solid Tumor antigen** | **Disease tumor** |
|---|---|
| PRLR | Breast Cancer |
| CLCA1 | colorectal Cancer |
| MUC12 | colorectal Cancer |
| GUCY2C | colorectal Cancer |
| GPR35 | colorectal Cancer |
| CR1L | Gastric Cancer |
| MUC 17 | Gastric Cancer |
| TMPRSS11B | esophageal Cancer |
| MUC21 | esophageal Cancer |
| TMPRSS11E | esophageal Cancer |
| CD207 | bladder Cancer |
| SLC30A8 | pancreatic Cancer |
| CFC1 | pancreatic Cancer |
| SLC12A3 | Cervical Cancer |
| SSTR1 | Cervical tumor |
| GPR27 | Ovary tumor |
| FZD10 | Ovary tumor |
| TSHR | Thyroid Tumor |
| SIGLEC15 | Urothelial cancer |
| SLC6A3 | Renal cancer |
| KISS1R | Renal cancer |
| QRFPR | Renal cancer: |
| GPR119 | Pancreatic cancer |
| CLDN6 | Endometrial cancer/ Urothelial cancer |
| UPK2 | Urothelial cancer (including bladder cancer) |
| ADAM 12 | Breast cancer, pancreatic cancer and the like |
| SLC45A3 | Prostate cancer |
| ACPP | Prostate cancer |
| MUC21 | Esophageal cancer |
| MUC16 | Ovarian cancer |
| MS4A12 | Colorectal cancer |
| ALPP | Endometrial cancer |
| CEA | Colorectal carcinoma |
| EphA2 | Glioma |
| FAP | Mesothelioma |
| GPC3 | Lung squamous cell carcinoma |
| IL13-Rα2 | Glioma |
| Mesothelin | Metastatic cancer |
| PSMA | Prostate cancer |
| ROR1 | Breast lung carcinoma |
| VEGFR-II | Metastatic cancer |
| GD2 | Neuroblastoma |
| FR-α | Ovarian carcinoma |
| ErbB2 | Carcinomas |
| EpCAM | Carcinomas |
| EGFRvIII | Glioma-Glioblastoma |
| EGFR | Glioma-NSCL cancer |
| tMUC 1 | Cholangiocarcinoma, Pancreatic cancer, Breast |
| PSCA | pancreas, stomach, or prostate cancer |

Throughout this specification, unless the context requires otherwise, the words "comprise," "includes" and "including" will be understood to imply the inclusion of a stated step or element (ingredients or components) or group of steps or elements (ingredients or components) but not the exclusion of any other step or element or group of steps or elements.

The phrase "consisting of" is meant to include, and is limited to, whatever follows the phrase "consisting of." Thus, the phrase "consisting of" indicates that the listed elements are required or mandatory and that no other elements may be present.

The phrase "consisting essentially of" is meant to include any element listed after the phrase and can include other elements that do not interfere with or contribute to the activity or action specified in the disclosure for the listed elements. Thus, the phrase "consisting essentially of" indicates that the listed elements are required or mandatory, but that other elements are optional and may or may not be present depending upon whether or not they affect the activity or action of the listed elements.

The terms "complementary" and "complementarity" refer to polynucleotides (i.e., a sequence of nucleotides) related by the base-pairing rules. For example, the sequence "A-G-T," is complementary to the sequence "T-C-A." Complementarity may be "partial," in which only some of the nucleic acids' bases are matched according to the base pairing rules or there may be "complete" or "total" complementarity between the nucleic acids. The degree of complementarity between nucleic acid strands has significant effects on the efficiency and strength of hybridization between nucleic acid strands.

The term "corresponds to" or "corresponding to" refers to (a) a polynucleotide having a nucleotide sequence that is substantially identical or complementary to all or a portion of a reference polynucleotide sequence or encoding an amino acid sequence identical to an amino acid sequence in a peptide or protein; or (b) a peptide or polypeptide having an amino acid sequence that is substantially identical to a sequence of amino acids in a reference peptide or protein.

The term "costimulatory ligand" refers to a molecule on an antigen presenting cell (e.g., an APC, dendritic cell, B cell, and the like) that specifically binds a cognate costimulatory molecule on a T cell, thereby providing a signal which, in addition to the primary signal provided by, for instance, binding of a TCR/CD3 complex with an MHC molecule loaded with peptide, mediates a T cell response, including at least one of proliferation, activation, differentiation, and other cellular responses. A costimulatory ligand can include B7-1 (CD80), B7-2 (CD86), PD-L1, PD-L2, 4-1BBL, OX40L, inducible co-stimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, a ligand for CD7, an agonist or antibody that binds the Toll ligand receptor and a ligand that specifically binds with B7-H3. A co-stimulatory ligand also includes, inter alia, an agonist or an antibody that specifically binds with a co-stimulatory molecule present on a T cell, such as CD27, CD28, 4-1BB, OX40, CD30, CD40, PD-1, ICOS, lymphocyte function-associated antigen-1 (LFA-1), CD2, CD7, LIGHT, NKG2C, B7-H3, and a ligand that specifically binds CD83.

The term "co-stimulatory molecule" refers to the cognate binding partner on a T cell that specifically binds with a co-stimulatory ligand, thereby mediating a co-stimulatory response by the T cell, such as proliferation. Co-stimulatory molecules include an MHC class I molecule, BTLA, and a Toll-like receptor.

The term "co-stimulatory signal" refers to a signal, which in combination with a primary signal, such as TCR/CD3 ligation, leads to T cell proliferation and/or upregulation or downregulation of key molecules. The terms "disease" and "condition" may be used interchangeably or may be different in that the particular malady or condition may not have a known causative agent (so that etiology has not yet been worked out), and it is therefore not yet recognized as a disease but only as an undesirable condition or syndrome, wherein a more or less specific set of symptoms have been identified by clinicians. The term "disease" is a state of health of a subject wherein the subject cannot maintain homeostasis, and wherein if the disease is not ameliorated then the subject's health continues to deteriorate. In contrast, a "disorder" in a subject is a state of health in which the animal is able to maintain homeostasis, but in which the animal's state of health is less favorable than it would be in the absence of the disorder. Left untreated, a disorder does not necessarily cause a further decrease in the animal's state of health.

The term "effective" refers to adequate to accomplish a desired, expected, or intended result. For example, an "effective amount" in the context of treatment may be an amount of a compound sufficient to produce a therapeutic or prophylactic benefit.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence (except that a "T" is replaced by a "U") and is usually provided in sequence listings, and the non-coding strand, used as the template for transcription of a gene or cDNA, can be referred to as encoding the protein or other product of that gene or cDNA.

The term "exogenous" refers to a molecule that does not naturally occur in a wild-type cell or organism but is typically introduced into the cell by molecular biological techniques. Examples of exogenous polynucleotides include vectors, plasmids, and/or man-made nucleic acid constructs encoding the desired protein. With regard to polynucleotides and proteins, the term "endogenous" or "native" refers to naturally-occurring polynucleotide or amino acid sequences that may be found in a given wild-type cell or organism. Also, a particular polynucleotide sequence that is isolated from a first organism and transferred to a second organism by molecular biological techniques is typically considered an "exogenous" polynucleotide or amino acid sequence with respect to the second organism. In specific embodiments, polynucleotide sequences can be "introduced" by molecular biological techniques into a microorganism that already contains such a polynucleotide sequence, for instance, to create one or more additional copies of an otherwise naturally-occurring polynucleotide sequence, and thereby facilitate overexpression of the encoded polypeptide.

The term "expression" refers to the transcription and/or translation of a particular nucleotide sequence driven by its promoter.

The term "expression vector" refers to a vector including a recombinant polynucleotide including expression control sequences operably linked to a nucleotide sequence to be expressed. An expression vector includes sufficient cis-acting elements for expression; other elements for expression can be supplied by the host cell or in an *in vitro* expression system. Expression vectors include all those known in the art, such as cosmids, plasmids (e.g., naked or contained in liposomes) and viruses (e.g., lentiviruses, retroviruses, adenoviruses, and adeno-associated viruses) that incorporate the recombinant polynucleotide.

The term "homologous" refers to sequence similarity or sequence identity between two polypeptides or between two polynucleotides when a position in both of the two compared sequences is occupied by the same base or amino acid monomer subunit, e.g., if a position in each of two DNA molecules is occupied by adenine, then the molecules are homologous at that position. The percent of homology between two sequences is a function of the number of matching or homologous positions shared by the two sequences divided by the number of positions compared ×100. For example, if 6 of 10 of the positions in two sequences are matched or homologous then the two sequences are 60% homologous. By way of example, the DNA sequences ATTGCC and TATGGC share 50% homology. A comparison is made when two sequences are aligned to give maximum homology.

The term "immunoglobulin" or "Ig," refers to a class of proteins, which function as antibodies. The five members included in this class of proteins are IgA, IgG, IgM, IgD, and IgE. IgA is the primary antibody that is present in body secretions, such as saliva, tears, breast milk, gastrointestinal secretions and mucus secretions of the respiratory and genitourinary tracts. IgG is the most common circulating antibody. IgM is the main immunoglobulin produced in the primary immune response in most subjects. It is the most efficient immunoglobulin in agglutination, complement fixation, and other antibody responses, and is important in defense against bacteria and viruses. IgD is the immunoglobulin that has no known antibody function but may serve as an antigen receptor. IgE is the immunoglobulin that mediates immediate hypersensitivity by causing the release of mediators from mast cells and basophils upon exposure to the allergen.

The term "isolated" refers to a material that is substantially or essentially free from components that normally accompany it in its native state. The material can be a cell or a macromolecule such as a protein or nucleic acid. For example, an "isolated polynucleotide," as used herein, refers to a polynucleotide, which has been purified from the sequences which flank it in a naturally-occurring state, e.g., a DNA fragment which has been removed from the sequences that are normally adjacent to the fragment. Alternatively, an "isolated peptide" or an "isolated polypeptide" and the like, as used herein, refer to *in vitro* isolation and/or purification of a peptide or polypeptide molecule from its natural cellular environment, and from association with other components of the cell.

The term "substantially purified" refers to a material that is substantially frr from components that normally associated with it in its native state. For example, a substantially purified cell refers to a cell that has been separated from other cell types with which it is normally associated in its naturally occurring or native state. In some instances, a population of substantially purified cells refers to a homogenous population of cells. In other instances, this term refers simply to a cell that has been separated from the cells with which they are naturally associated in their natural state. In embodiments, the cells are cultured *in vitro.* In embodiments, the cells are not cultured *in vitro.*

In the context of the present disclosure, the following abbreviations for the commonly occurring nucleic acid bases are used. "A" refers to adenosine, "C" refers to cytosine, "G" refers to guanosine, "T" refers to thymidine, and "U" refers to uridine.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or an RNA may also include introns to the extent that the nucleotide sequence encoding the protein may in some version contain an intron(s).

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. Moreover, the use of lentiviruses enables integration of the genetic information into the host chromosome resulting in stably transduced genetic information. HIV, SIV, and FIV are all examples of lentiviruses. Vectors derived from lentiviruses offer the means to achieve significant levels of gene transfer *in vivo.*

The term "modulating," refers to mediating a detectable increase or decrease in the level of a response in a subject compared with the level of a response in the subject in the absence of a treatment or compound, and/or compared with the level of a response in an otherwise identical but untreated subject. The term encompasses perturbing and/or affecting a native signal or response thereby mediating a beneficial therapeutic response in a subject, preferably, a human.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation.

The term "under transcriptional control" refers to a promoter being operably linked to and in the correct location and orientation in relation to a polynucleotide to control the initiation of transcription by RNA polymerase and expression of the polynucleotide.

The term "overexpressed" tumor antigen or "overexpression" of the tumor antigen is intended to indicate an abnormal level of expression of the tumor antigen in a cell from a disease area such as a solid tumor within a specific tissue or organ of the patient relative to the level of expression in a normal cell from that tissue or organ. Patients having solid tumors or a hematological malignancy characterized by overexpression of the tumor antigen can be determined by standard assays known in the art.

The term "parenteral administration" of a composition includes, e.g., subcutaneous (s.c.), intravenous (i.v.), intramuscular (i.m.), intrasternal injection, or infusion techniques.

The terms "patient," "subject," and "individual," and the like are used interchangeably herein, and refer to any human, animal, or living organism, amenable to the methods described herein. In certain non-limiting examples, the patient, subject, or individual is a human or animal. In examples, the term "subject" is intended to include living organisms in which an immune response can be elicited (e.g., mammals). Examples of subjects include humans, and animals such as dogs, cats, mice, rats, and non-human transgenic species thereof.

A subject in need of treatment or in need thereof includes a subject having a disease, condition, or disorder that needs to be treated. A subject in need thereof also includes a subject that needs treatment for prevention of a disease, condition, or disorder. In some examples, the disease is cancer.

The term "polynucleotide" or "nucleic acid" refers to mRNA, RNA, cRNA, rRNA, cDNA or DNA. The term typically refers to a polymeric form of nucleotides of at least 10 bases in length, either ribonucleotides or deoxynucleotides or a modified form of either type of nucleotide. The term includes all forms of nucleic acids including single and double stranded forms of nucleic acids.

The terms "polynucleotide variant" and "variant" and the like refer to polynucleotides displaying substantial sequence identity with a reference polynucleotide sequence or polynucleotides that hybridize with a reference sequence under stringent conditions that are defined hereinafter. These terms also encompass polynucleotides that are distinguished from a reference polynucleotide by the addition, deletion or substitution of at least one nucleotide. Accordingly, the terms "polynucleotide variant" and "variant" include polynucleotides in which one or more nucleotides have been added or deleted or replaced with different nucleotides. In this regard, it is well understood in the art that certain alterations inclusive of mutations, additions, deletions, and substitutions can be made to a reference polynucleotide whereby the altered polynucleotide retains the biological function or activity of the reference polynucleotide or has increased activity in relation to the reference polynucleotide (i.e., optimized). Polynucleotide variants include, for example, polynucleotides having at least 50% (and at least 51% to at least 99% and all integer percentages in between, e.g., 90%, 95%, or 98%) sequence identity with a reference polynucleotide sequence described herein. The terms "polynucleotide variant" and "variant" also include naturally-occurring allelic variants and orthologs.

The terms "polypeptide," "polypeptide fragment," "peptide," and "protein" are used interchangeably herein to refer to a polymer of amino acid residues and to variants and synthetic analogues of the same. Thus, these terms apply to amino acid polymers in which one or more amino acid residues are synthetic non-naturally occurring amino acids, such as a chemical analogue of a corresponding naturally occurring amino acid, as well as to naturally-occurring amino acid polymers. In certain aspects, polypeptides may include enzymatic polypeptides, or "enzymes," which typically catalyze (i.e., increase the rate of) various chemical reactions.

The term "polypeptide variant" refers to polypeptides that are distinguished from a reference polypeptide sequence by the addition, deletion, or substitution of at least one amino acid residue. In some examples, a polypeptide variant is distinguished from a reference polypeptide by one or more substitutions, which may be conservative or non-conservative. In some examples, the polypeptide variant comprises conservative substitutions and, in this regard, it is well understood in the art that some amino acids may be changed to others with broadly similar properties without changing the nature of the activity of the polypeptide. Polypeptide variants also encompass polypeptides in which one or more amino acids have been added or deleted or replaced with different amino acid residues.

The term "promoter" refers to a DNA sequence recognized by the synthetic machinery of the cell or introduced synthetic machinery, required to initiate the specific transcription of a polynucleotide sequence. The term "expression control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

The term "bind," "binds," or "interacts with" refers to a molecule recognizing and adhering to a second molecule in a sample or organism but does not substantially recognize or adhere to other structurally unrelated molecules in the sample. The term "specifically binds," as used herein with respect to an antibody, refers to an antibody which recognizes a specific antigen, but does not substantially recognize or bind other molecules in a sample. For example, an antibody that specifically binds an antigen from one species may also bind that antigen from one or more species. But, such cross-species reactivity does not itself alter the classification of an antibody as specific. In another example, an antibody that specifically binds an antigen may also bind different allelic forms of the antigen. However, such cross reactivity does not itself alter the classification of an antibody as specific. In some instances, the terms "specific binding" or "specifically binding," can be used in reference to the interaction of an antibody, a protein, or a peptide with a second chemical species, to mean that the interaction is dependent upon the presence of a particular structure (e.g., an antigenic determinant or epitope) on the chemical species; for example, an antibody recognizes and binds a specific protein structure rather than to any protein. If an antibody is specific for epitope "A," the presence of a molecule containing epitope A (or free, unlabeled A), in a reaction containing labeled "A" and the antibody, will reduce the amount of labeled A bound to the antibody.

A "binding protein" is a protein that is able to bind non-covalently to another molecule. A binding protein can bind to, for example, a DNA molecule (a DNA-binding protein), an RNA molecule (an RNA-binding protein) and/or a protein molecule (a protein-binding protein). In the case of a protein-binding protein, it can bind to itself (to form homodimers, homotrimers, etc.) and/or it can bind to one or more molecules of a different protein or proteins. A binding protein can have more than one type of binding activity. For example, zinc finger proteins have DNA-binding, RNA-binding, and protein-binding activity.

A "zinc finger DNA binding protein" (or binding domain) is a protein, or a domain within a larger protein, that binds DNA in a sequence-specific manner through one or more zinc fingers, which are regions of amino acid sequence within the binding domain whose structure is stabilized through coordination of a zinc ion. The term zinc finger DNA binding protein is often abbreviated as zinc finger protein or ZFP.

Zinc finger binding domains can be "engineered" to bind to a predetermined nucleotide sequence, for example via engineering (altering one or more amino acids) of the recognition helix region of a naturally occurring zinc finger protein. Further, a Zinc finger binding domain may be fused a DNA-cleavage domain to form a Zinc finger nuclease (ZFN) targeting a specific desired DNA sequence. For example, a pair of ZFNs (e.g., a ZFN-left arm and a ZFN-right arm) may be engineered to target and cause modifications of specific desired DNA sequences (e.g., TRAC genes).

"Cleavage" refers to the breakage of the covalent backbone of a DNA molecule. Cleavage can be initiated by a variety of methods including, but not limited to, enzymatic or chemical hydrolysis of a phosphodiester bond. Both single-stranded cleavage and double-stranded cleavage are possible, and double-stranded cleavage can occur as a result of two distinct single-stranded cleavage events. DNA cleavage can result in the production of either blunt ends or staggered ends. In some examples, fusion polypeptides are used for targeted double-stranded DNA cleavage.

A "target site" or "target sequence" is a nucleic acid sequence that defines a portion of a nucleic acid to which a binding molecule will bind, provided sufficient conditions for binding exist. For example, the sequence 5' GAATTC 3' is a target site for the Eco RI restriction endonuclease.

A "fusion" molecule is a molecule in which two or more subunit molecules are linked, preferably covalently. The subunit molecules can be the same chemical type of molecule or can be different chemical types of molecules. Examples of the first type of fusion molecule include, but are not limited to, fusion proteins (for example, a fusion between a ZFP DNA-binding domain and one or more activation domains) and fusion nucleic acids (for example, a nucleic acid encoding the fusion protein described supra). Examples of the second type of fusion molecule include, but are not limited to, a fusion between a triplex-forming nucleic acid and a polypeptide, and a fusion between a minor groove binder and a nucleic acid.

Expression of a fusion protein in a cell can result from delivery of the fusion protein to the cell or by delivery of a polynucleotide encoding the fusion protein to a cell, wherein the polynucleotide is transcribed, and the transcript is translated, to generate the fusion protein. Trans-splicing, polypeptide cleavage, and polypeptide ligation can also be involved in the expression of the protein in a cell. Methods for polynucleotide and polypeptide delivery to cells are presented elsewhere in this disclosure.

"Modulation" of gene expression refers to a change in the activity of a gene. Modulation of expression can include but is not limited to, gene activation and gene repression. Genome editing (e.g., cleavage, alteration, inactivation, random mutation) can be used to modulate expression. Gene inactivation refers to any reduction in gene expression as compared to a cell that does not include a ZFP as described herein. Thus, gene inactivation may be partial or complete.

A "region of interest" is any region of cellular chromatin, such as, for example, a gene or a non-coding sequence within or adjacent to a gene, in which it is desirable to bind an exogenous molecule. Binding can be for the purposes of targeted DNA cleavage and/or targeted recombination. A region of interest can be present in a chromosome, an episome, an organellar genome (e.g., mitochondrial, chloroplast), or an infecting viral genome, for example. A region of interest can be within the coding region of a gene, within transcribed non-coding regions such as, for example, leader sequences, trailer sequences or introns, or within non-transcribed regions, either upstream or downstream of the coding region. A region of interest can be as small as a single nucleotide pair or up to 2,000 nucleotide pairs in length, or any integral value of nucleotide pairs.

By "statistically significant," it is meant that the result was unlikely to have occurred by chance. Statistical significance can be determined by any method known in the art. Commonly used measures of significance include the p-value, which is the frequency or probability with which the observed event would occur if the null hypothesis were true. If the obtained p-value is smaller than the significance level, then the null hypothesis is rejected. In simple cases, the significance level is defined at a p-value of 0.05 or less. A "decreased" or "reduced" or "lesser" amount is typically a "statistically significant" or a physiologically significant amount, and may include a decrease that is about 1.1, 1.2, 1.3, 1.4, 1.5, 1.6 1.7, 1.8, 1.9, 2, 2.5, 3, 3.5, 4, 4.5, 5, 6, 7, 8, 9, 10, 15, 20, 30, 40, or 50 or more times (*e.g.,* 100, 500, 1000 times) (including all integers and decimal points in between and above 1, *e.g.,* 1.5, 1.6, 1.7. 1.8, etc.) an amount or level described herein.

The term "stimulation," refers to a primary response induced by binding of a stimulatory molecule (e.g., a TCR/CD3 complex) with its cognate ligand thereby mediating a signal transduction event, such as signal transduction via the TCR/CD3 complex. Stimulation can mediate altered expression of certain molecules, such as downregulation of TGF-β, and/or reorganization of cytoskeletal structures. CD3 zeta is not the only suitable primary signaling domain for a CAR construct with respect to the primary response. For example, back in 1993, both CD3 zeta and FcR gamma were shown as functional primary signaling domains of CAR molecules. Eshhar et al., "Specific activation and targeting of cytotoxic lymphocytes through chimeric single chains consisting of antibody-binding domains and the gamma or zeta subunits of the immunoglobulin and T cell receptors" PNAS, 1993 Jan 15;90(2):720-4, showed that two CAR constructs in which an scFv was fused to "either the FcR gamma chain or the CD3 complex chain" triggered T cell activation and target cell. Notably, as demonstrated in Eshhar et al., CAR constructs containing only the primary signaling domain CD3 zeta or FcR gamma are functional without the co-presence of co-stimulatory domains. Additional non-CD3 zeta based CAR constructs have been developed over the years. For example, Wang et al. (,"A Chimeric Antigen Receptor (CARs) Based Upon a Killer Immunoglobulin-Like Receptor (KIR) Triggers Robust Cytotoxic Activity in Solid Tumors" Molecular Therapy, vol. 22, no. Suppl.1, May 2014, page S57) tested a CAR molecule in which an scFv was fused to "the transmembrane and cytoplasmic domain of' a killer immunoglobulin-like receptor (KIR). Wang et al. reported that, "a KIR-based CAR targeting mesothelin (SS 1-KIR) triggers antigen-specific cytotoxic activity and cytokine production that is comparable to CD3--based CARs." A second publication from the same group, Wang et al. ("Generation of Potent T-cell Immunotherapy for Cancer Using DAP12-Based, Multichain, Chimeric Immunoreceptors" Cancer Immunol Res. 2015 Jul;3(7):815-26) showed that a CAR molecule in which "a single-chain variable fragment for antigen recognition was fused to the transmembrane and cytoplasmic domains of KIR2DS2, a stimulatory killer immunoglobulin-like receptor (KIR)" functioned both in vitro and in vivo "when introduced into human T cells with DAP12, an immunotyrosine-based activation motifs-containing adaptor."

The term "stimulatory molecule" refers to a molecule on a T cell that specifically binds a cognate stimulatory ligand present on an antigen presenting cell. For example, a functional signaling domain derived from a stimulatory molecule is the zeta chain associated with the T cell receptor complex.

The term "stimulatory ligand" refers to a ligand that when present on an antigen presenting cell (e.g., an APC, a dendritic cell, a B-cell, and the like.) can specifically bind with a cognate binding partner (referred to herein as a "stimulatory molecule") on a cell, for example a T cell, thereby mediating a primary response by the T cell, including activation, initiation of an immune response, proliferation, and similar processes. Stimulatory ligands are well-known in the art and encompass, inter alia, an MHC Class I molecule loaded with a peptide, an anti-CD3 antibody, a superagonist anti-CD28 antibody, and a superagonist anti-CD2 antibody.

The term "therapeutic" refers to a treatment and/or prophylaxis. A therapeutic effect is obtained by suppression, remission, or eradication of a disease state or alleviating the symptoms of a disease state.

The term "therapeutically effective amount" refers to the amount of the subject compound that will elicit the biological or medical response of a tissue, system, or subject that is being sought by the researcher, veterinarian, medical doctor or another clinician. The term "therapeutically effective amount" includes that amount of a compound that, when administered, is sufficient to prevent the development of, or alleviate to some extent, one or more of the signs or symptoms of the disorder or disease being treated. The therapeutically effective amount will vary depending on the compound, the disease and its severity and the age, weight, etc., of the subject to be treated.

The term "treat a disease" refers to the reduction of the frequency or severity of at least one sign or symptom of a disease or disorder experienced by a subject.

The term "transfected" or "transformed" or "transduced" refers to a process by which an exogenous nucleic acid is transferred or introduced into the host cell. A "transfected" or "transformed" or "transduced" cell is one which has been transfected, transformed, or transduced with exogenous nucleic acid. The cell includes the primary subject cell and its progeny.

The term "vector" refers to a polynucleotide that comprises an isolated nucleic acid and which can be used to deliver the isolated nucleic acid to the interior of a cell in vitro and in vivo (in a subject). Numerous vectors are known in the art including linear polynucleotides, polynucleotides associated with ionic or amphiphilic compounds, plasmids, and viruses. Thus, the term "vector" includes an autonomously replicating plasmid or a virus. The term also includes non-plasmid and non-viral compounds which facilitate transfer of nucleic acid into cells, such as, for example, polylysine compounds, liposomes, and the like. Examples of viral vectors include, adenoviral vectors, adeno-associated virus vectors, retroviral vectors, and others. For example, lentiviruses are complex retroviruses, which, in addition to the common retroviral genes gag, pol, and env, contain other genes with regulatory or structural function. Lentiviral vectors are well known in the art. Some examples of lentivirus include the Human Immunodeficiency Viruses: HIV-1, HIV-2, and the Simian Immunodeficiency Virus: SIV. Lentiviral vectors have been generated by multiply attenuating the HIV virulence genes, for example, the genes env, vif, vpr, vpu, and nef are deleted making the vector biologically safe.

Ranges: throughout this disclosure, various aspects of the disclosure can be presented in a range format. It should be understood that the description in range format is merely for convenience and brevity and should not be construed as an inflexible limitation on the scope of the disclosure. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. This applies regardless of the breadth of the range.

Disclosed herein are methods relating to treating cancer using chimeric antigen receptor (CAR) cells. Also disclosed herein are isolated nucleic acids encoding a CAR, wherein the CAR comprises an extracellular domain, a transmembrane domain, and an intracellular domain, wherein the extracellular domain of the CAR binds an antigen of a solid tumor. In the invention the CAR comprises an antibody binding domain that comprises: a heavy chain variable region (HVR) comprising amino acid sequence SEQ ID NO: 83 and a light chain variable region (LVR) comprising amino acid sequence SEQ ID NO: 82. For example, transcriptional data shows that expression of antigens such as SLC6A3, KISS1R, QRFPR in normal tissues is very low, but expression of such antigens in cells related to renal cancer is high. Information of some of the antigens is provided below in Table 2.

**Table 2**

| Gene name | Subcellular localization | Organ mainly expressing | Target Tumor | Target SEQ ID NO. |
|---|---|---|---|---|
| *SIGLEC15* | Plasma membrane | Expression in all normal tissues is very low | Urothelial cancer | 17 |
| *SLC6A3* | Plasma membrane | Expression in all normal tissues is very low | Renal cancer | 18 |
| *KISS1R* | Plasma membrane | Expression in all normal tissues is very low | Renal cancer | 19 |
| *QRFPR* | Plasma membrane | Expression in all normal tissues is very low | Renal cancer: | 20 |
| *GPR119* | Plasma membrane | Expression in all normal tissues is very low | Pancreatic cancer | 21 |
| *CLDN6* | Plasma membrane | Expression in all normal tissues is very low | Endometrial cancer/ Urothelial cancer | 22 |
| *UPK2* | Plasma membrane | Urethra / bladder | Urothelial cancer (including bladder cancer) | 1 |
| *ADAM12* | Plasma membrane | placenta | Breast cancer, pancreatic cancer and the like | 2 |
| *SLC45A3* | Plasma membrane | prostate | Prostate cancer | 3 |
| *ACPP* | Plasma membrane | prostate | Prostate cancer | 4 |
| *MUC21* | Plasma membrane | esophagus | Esophageal cancer | 5 |
| *MUC16* | Plasma membrane | Cervical / Fallopian tube | Ovarian cancer | 6 |
| *MS4A12* | Plasma membrane | the large intestine | Colorectal cancer | 7 |
| *ALPP* | Plasma membrane | Placenta / cervix | Endometrial cancer | 8 |
| *SLC2A 14* | Plasma membrane | testis | Testicular cancer | 9 |
| *GS1-259H13.2* | Plasma membrane | testis | Thyroid cancer or glioma or testicular cancer and other | 10 |
| *ERVFRD-1* | Plasma membrane | Placenta or parathyroid | Kidney cancer or Urethral cancer and many others | 11 |
| *ADGRG2* | Plasma membrane | Epididymis | Ovarian cancer | 12 |
| *ECEL1* | Plasma membrane | Ovary | Endometrial cancer | 13 |
| *CHRNA2* | Plasma membrane | Prostate or cortex | Prostate cancer | 14 |
| *GP2* | Plasma membrane | pancreas | Pancreatic cancer | 15 |
| *PSG9* | Plasma membrane | placenta | Kidney cancer or liver cancer | 16 |

In some disclosures, the extracellular domain of the CAR binds SIGLEC15. SIGLEC15 is a receptor protein expressed on the cell membrane, which recognizes sialylated glycans. Transcriptional data predict that it is overexpressed in urothelial cancer cells and is expressed at a low level in normal tissues. It is mainly found in spleen and lymph nodes, and other immune organs have a certain amount of low expression. For example, the extracellular domain of the CAR binds SIGLEC15 having the amino acid sequence of SEQ ID NO: 17. In some examples, the extracellular domain comprises one of the amino acid sequences of SEQ ID NOs: 45-56. Some disclosures relate to a method of eliciting and/or enhancing T cell response in a subject having the solid tumor or treating the solid tumor of the subject, the method comprising administering an effective amount of T cell comprising the CAR to the subject. In some disclosures, the tumor is associated with urothelial cancer.

The T cell response in a subject refers to cell-mediated immunity associated with a helper, killer, regulatory, and other types T cells. For example, T cell response may include activities such as assistance to other white blood cells in immunologic processes and identifying and destroying virus-infected cells and tumor cells. T cell response in the subject may be measured via various indicators such as a number of virus-infected cells and /or tumor cells that T cells kill, an amount of cytokines that T cells release in co-culturing with virus-infected cells and/or tumor cells, a level of proliferation of T cells in the subject, a phenotype change of T cells (e.g., changes to memory T cells), and a level longevity or lifetime of T cells in the subject.

In vitro killing assay may be performed by measuring the killing efficacy of CAR T cells by co-culturing CAR T cells with antigen-positive cells. CAR T cells may be considered to have killing effect on the corresponding antigen-positive cells by showing a decrease in the number of corresponding antigen-positive cells co-cultured with CAR T cells and an increase in the release of IFNγ, TNFα, etc. as compared to control cells that do not express the corresponding antigen. Further, in vivo antitumor activity of the CAR T cells may be tested. For example, xenograft models may be established using the antigens described herein in immunodeficient mice. Heterotransplantation of human cancer cells or tumor biopsies into immunodeficient rodents (xenograft models) has, for the past two decades, constituted the major preclinical screen for the development of novel cancer therapeutics (Song et al., Cancer Res. PMC 2014 Aug 21, and Morton et al., Nature Protocols, 2, -247 - 250 (2007)). To evaluate the anti-tumor activity of CAR T cells in vivo, immunodeficient mice bearing tumor xenografts were evaluated for CAR T cell anti-tumor activity (e.g., a decrease in mouse tumors and mouse blood IFNγ, TNFα, et al.).

The term "chimeric antigen receptor" or alternatively a "CAR" refers to a recombinant polypeptide construct comprising at least an extracellular antigen binding domain, a transmembrane domain and an intracellular signaling domain (e.g., cytoplasmic domain). In embodiments, the domains in the CAR polypeptide construct are on the same polypeptide chain (e.g., comprising a chimeric fusion protein). In embodiments, the domains of the CAR polypeptide are not on the same molecule, e.g. not contiguous with each other or are on different polypeptide chains.

In embodiments, the intracellular signaling domain may include a functional signaling domain derived from a stimulatory molecule and/or a co-stimulatory molecule as described herein. In embodiments, the intracellular signaling domain includes a functional signaling domain derived from a primary signaling domain (e.g., a primary signaling domain of CD3-zeta). In embodiments, the intracellular signaling domain further includes one or more functional signaling domains derived from at least one co-stimulatory molecule. The co-stimulatory signaling region refers to a portion of the CAR including the intracellular domain of a co-stimulatory molecule. Co-stimulatory molecules can include cell surface molecules for inducing an efficient response from the lymphocytes (in response to an antigen).

Between the extracellular domain and the transmembrane domain of the CAR, there may be incorporated a spacer domain. As used herein, the term "spacer domain" generally means any oligo- or polypeptide that functions to link the transmembrane domain to the extracellular domain and/or the cytoplasmic domain in the polypeptide chain. A spacer domain may include up to 300 amino acids, 10 to 100 amino acids, or 25 to 50 amino acids.

In some disclosures, the extracellular domain of a CAR may include an antigen binding domain (e.g., a scFv, a single domain antibody, or TCR, such as a TCR alpha binding domain or a TCR beta binding domain) that targets a specific tumor marker (e.g., a tumor antigen). Tumor antigens are proteins that are produced by tumor cells that elicit an immune response, particularly T cell mediated immune responses. Tumor antigens are well known in the art and include, for example, a glioma-associated antigen, carcinoembryonic antigen (CEA), β-human chorionic gonadotropin, alphafetoprotein (AFP), lectin-reactive AFP, thyroglobulin, RAGE-1, MN-CA IX, human telomerase reverse transcriptase, RU1, RU2 (AS), intestinal carboxyl esterase, mut hsp70-2, M-CSF, prostase, prostate-specific antigen (PSA), PAP, NY-ESO-1, LAGE-1a, p53, prostein, PSMA, Her2/neu, survivin and telomerase, prostate-carcinoma tumor antigen-1 (PCTA-1), MAGE, ELF2M, neutrophil elastase, ephrinB2, CD22, insulin growth factor (IGF)-I, IGF-II, IGF-I receptor and mesothelin. For example, when the antigen that the CAR binds is CD19, the CAR thereof is referred to as CD19CAR.

In some embodiments of the invention, the extracellular ligand-binding domain comprises a scFv comprising a heavy chain variable region (HVR) comprising amino acid sequence SEQ ID NO: 83 and a light chain variable region (LVR) comprising amino acid sequence SEQ ID NO: 82. joined by a flexible linker. Single chain variable region fragments are made by linking light and/or heavy chain variable regions by using a short linking peptide (Bird et al., Science 242:423-426, 1988). An example of a linking peptide is the GS linker having the amino acid sequence (GGGGS)3 (SEQ ID: 24), which bridges approximately 3.5 nm between the carboxy terminus of one variable region and the amino terminus of the other variable region. Linkers of other sequences have been designed and used (Bird et al., 1988, supra). In general, linkers can be short, flexible polypeptides comprising about 20 or fewer amino acid residues. Linkers can in turn be modified for additional functions, such as attachment of drugs or attachment to solid supports. The single chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect or mammalian cells, or prokaryotic, such as E. coli. Polynucleotides encoding the scFv of interest can be made by routine manipulations such as ligation of polynucleotides. The resultant scFv can be isolated using standard protein purification techniques known in the art.

In some disclosures, the tumor antigen includes HER2, CD19, CD20, CD22, Kappa or light chain, CD30, CD33, CD123, CD38, ROR1, ErbB3/4, EGFR, EGFRvIII, EphA2, FAP, carcinoembryonic antigen, EGP2, EGP40, mesothelin, TAG72, PSMA, NKG2D ligands, B7-H6, IL-13 receptor α 2, IL-11 receptor α, MUC1, MUC16, CA9, GD2, GD3, HMW-MAA, CD171, Lewis Y, G250/CAIX, HLA-Al MAGE A1, HLA-A2 NY-ESO-1, PSC1, folate receptor-a, CD44v7/8, 8H9, NCAM, VEGF receptors, 5T4, Fetal AchR, NKG2D ligands, CD44v6, TEM1, TEM8, or viral-associated antigens expressed by a tumor. In some disclosures, the binding element of the CAR may include any antigen binding moiety that when bound to its cognate antigen, affects a tumor cell such that the tumor cell fails to grow, or is promoted to die or diminish.

In some disclosures, the extracellular domain of the CAR binds KISS1R. KISS1R is a galanin-like G protein-coupled receptor that binds Kisspeptin (metastin), a peptide encoded by the metastasis suppressor gene KISS1. KISS1 R may be involved in the regulation of endocrine function. For example, the extracellular domain of the CAR binds KISS1R having the amino acid sequence of SEQ ID NO: 19. In some disclosures, the extracellular domain of the CAR comprises one of the amino acid sequences of SEQ ID NOs: 71 and 72. Some disclosures relate to a method of eliciting and/or enhancing T cell response in a subject having a solid tumor or treating a solid tumor in the subject, wherein the method comprises administering an effective amount of T cells comprising the CAR. In Some disclosures, the solid tumor is associated with renal cancer.

In some disclosures, the extracellular domain of the CAR binds CLDN6. CLDN6 is a component of tight junction strands, which is a member of the claudin family, an integral membrane protein. Transcriptional data predict high expression in endometrial cancer, urothelial cancer, whereas expression in normal tissues is a component of tight junction strands, which are members of the claudin family Low volume. For example, the extracellular domain of the CAR binds CLDN6 having the amino acid sequence of SEQ ID NO: 22. In some disclosures, the extracellular domain of the CAR comprises one of the amino acid sequences of SEQ ID NOs: 29-44. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with endometrial cancer and/or urothelial cancer.

In some disclosures, the extracellular domain of the CAR bindsMUC16. MUC21 and MUC16 are large membrane-bound glycoproteins, which belong to mucin family. Mucins are O-glycosylated proteins that play an essential role in forming protective mucous barriers on epithelial surfaces. MUC21 has restricted expression toward esophagus, for esophageal cancer. MUC16 has low expression in normal tissues and low expression in the endometrium. In ovarian cancer, MUC16 is highly expressed. For example, the extracellular domain of the CAR binds MUC16 having the amino acid sequence of SEQ ID NO: 6. In some disclosures, the extracellular domain of the CAR comprises one of the amino acid sequences of SEQ ID NOs: 63-70. Some disclosures relate to a method of eliciting and/or enhancing T cell response in a subject having a solid tumor or treating a solid tumor in the subject, wherein the method comprises administering an effective amount of T cells comprising the CAR. In some disclosures, the solid tumor is associated with ovarian cancer.

In some disclosures, the extracellular domain of the CAR binds SLC6A3. SLC6A3 is a dopamine transporter, a member of the sodium- and chloride-dependent neurotransmitter transporter family. For example, the extracellular domain of the CAR binds SLC6A3 having the amino acid sequence of SEQ ID NO: 18. Some disclosures include a method of eliciting and/or enhancing T- cell response in a subject having a solid tumor or treating a solid tumor in the subject, wherein the method comprises administering an effective amount of T cells comprising the CAR. In some disclosures, the tumor comprises renal cancer.

In some disclosures, the extracellular domain of the CAR binds QRFPR. QRFPR is pyroglutamylated RFamide peptide receptor and may be involved in adipogenesis with its ligand, QRFP. For example, the extracellular domain of the CAR binds QRFPR having the amino acid sequence of SEQ ID NO: 20. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with renal cancer.

In some disclosures, the extracellular domain of the CAR binds GPR119. GPR119 is a member of the rhodopsin subfamily of G-protein-coupled receptors, has low expression in the pancreas and gastrointestinal tract, and may be involved in glucose homeostasis. Transcriptional data predict high expression in pancreatic cancer. For example, the extracellular domain of the CAR binds GPR119 having the amino acid sequence of SEQ ID NO: 21. Some disclosures relate to a method of eliciting and/or enhancing T- cell response in a subject having a solid tumor or treating a solid tumor of the subject, wherein the method comprises administering an effective amount of T cells comprising the CAR. In some disclosures, the tumor is associated with pancreatic cancer.

In some disclosures, the extracellular domain of the CAR bindsUPK2. UPK2 is one of the proteins of the highly conserved urothelium-specific integral membrane proteins of the asymmetric unit membrane, expressed primarily in urinary bladder in normal tissues and urothelial carcinoma, including bladder cancer. For example, the extracellular domain of the CAR binds UPK2 having the amino acid sequence of SEQ ID NO: 1. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with urothelial cancer and/or bladder cancer.

In some disclosures, the extracellular domain of the CAR binds ADAM12. ADAM12 is a member of a family of proteins that are structurally related to snake venom disintegrins, involved in cell-cell and cell-matrix interactions, and is highly expressed in tumors such as placenta and breast/pancreatic cancer. For example, the extracellular domain of the CAR binds ADAM12 having the amino acid sequence of SEQ ID NO: 2. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with breast cancer and/or pancreatic cancer.

In some disclosures, the extracellular domain of the CAR binds SLC45A3. SLC45A3 is a plasma membrane protein, normal tissue is mainly expressed in prostate, for prostate cancer. For example, the extracellular domain of the CAR binds SLC45A3 having the amino acid sequence of SEQ ID NO: 3. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with prostate cancer.

In the invention, the extracellular domain of the CAR binds ACPP. ACPP is an enzyme that catalyzes the conversion of orthophosphoric monoester to alcohol and orthophosphate, contains a transmembrane domain and localized in the plasma membrane-endosomal-lysosomal pathway. Normal tissue is specifically expressed in the prostate for prostate cancer. For example, the extracellular domain of the CAR binds ACPP having the amino acid sequence of SEQ ID NO: 4. Embodiments relate to a method of eliciting and/or enhancing T- cell response in a subject having a solid tumor or treating a solid tumor in the subject, wherein the method comprises administering an effective amount of T cells comprising the CAR. In embodiments, the solid tumor is associated with prostate cancer.

In some disclosures, the extracellular domain of the CAR binds MUC21. MUC21 and MUC16 are large membrane-bound glycoproteins, which belong to mucin family. Mucins are O-glycosylated proteins that play an essential role in forming protective mucous barriers on epithelial surfaces. MUC21 has restricted expression toward esophagus, when the subject has esophageal cancer. For example, the extracellular domain of the CAR binds MUC21 having the amino acid sequence of SEQ ID NO: 5. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with esophageal cancer.

In some disclosures, the extracellular domain of the CAR binds MS4A12. MS4A12 is a cell surface protein found in the apical membrane of colonocytes, restricted expression on colon, and may be used against colorectal cancer. For example, the extracellular domain of the CAR binds MS4A12 having the amino acid sequence of SEQ ID NO: 7. Some disclosures relate to a method of eliciting and/or enhancing T- cell response in a subject having a solid tumor or treating a solid tumor in the subject, wherein the method comprises administering an effective amount of T cells comprising the CAR. In some disclosures, the solid tumor is associated with colorectal cancer.

In some disclosures, the extracellular domain of the CAR binds ALPP. ALPP is an alkaline phosphatase, a metalloenzyme that catalyzes the hydrolysis of phosphoric acid monoesters., The expression of ALPP is restricted to the placenta, Strong ectopic expression of ALPP has been detected in ovarian adenocarcinoma, serous cystadenocarcinoma, and other ovarian cancer cells. For example, the extracellular domain of the CAR binds ALPP having the amino acid sequence of SEQ ID NO: 8. Some disclosures relate to a method of eliciting and/or enhancing T- cell response in a subject having a solid tumor or treating a solid tumor in the subject, wherein the method comprises administering an effective amount of T cells comprising he CAR. In some disclosures, the solid tumor is associated with endometrial cancer.

In some disclosures, the extracellular domain of the CAR binds SLC2A14. For example, the extracellular domain of the CAR binds SLC2A14 having the amino acid sequence of SEQ ID NO: 9. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with testicular cancer.

In some disclosures, the extracellular domain of the CAR binds GS1-259H13.2. For example, the extracellular domain of the CAR binds GS1-259H13.2 having the amino acid sequence of SEQ ID NO: 10. Some disclosures relate to a method of eliciting and/or enhancing T- cell response in a subject having a solid tumor or treating a solid tumor of the subject, wherein the method comprises administering an effective amount of T cells comprising the CAR. In some disclosures, the tumor is associated with thyroid cancer or glioma, or testicular cancer.

In some disclosures, the extracellular domain of the CAR binds ERVFRD-1. For example, the extracellular domain of the CAR binds ERVFRD-1 having the amino acid sequence of SEQ ID NO: 11. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with kidney cancer or Urethral cancer.

In some disclosures, the extracellular domain of the CAR binds ADGRG2. For example, the extracellular domain of the CAR binds ADGRG2 having the amino acid sequence of SEQ ID NO: 12. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with ovarian cancer.

In some disclosures, the extracellular domain of the CAR binds ECEL1. For example, the extracellular domain of the CAR binds ECEL1 having the amino acid sequence of SEQ ID NO: 13. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with endometrial cancer.

In some disclosures, the extracellular domain of the CAR binds CHRNA2. For example, the extracellular domain of the CAR binds CHRNA2 having the amino acid sequence of SEQ ID NO: 14. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with prostate cancer.

In some disclosures, the extracellular domain of the CAR binds GP2. For example, the extracellular domain of the CAR binds GP2 having the amino acid sequence of SEQ ID NO: 15. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with pancreatic cancer.

In some disclosures, the extracellular domain of the CAR binds PSG9. For example, the extracellular domain of the CAR binds PSG9 having the amino acid sequence of SEQ ID NO: 16. Some disclosures relate to a method of eliciting and/or enhancing T- cell response in a subject having a solid tumor or treating a solid tumor in the subject, wherein the method comprises administering an effective amount of T cells comprising the CAR. In some disclosures, the tumor is associated with Kidney cancer or liver cancer.

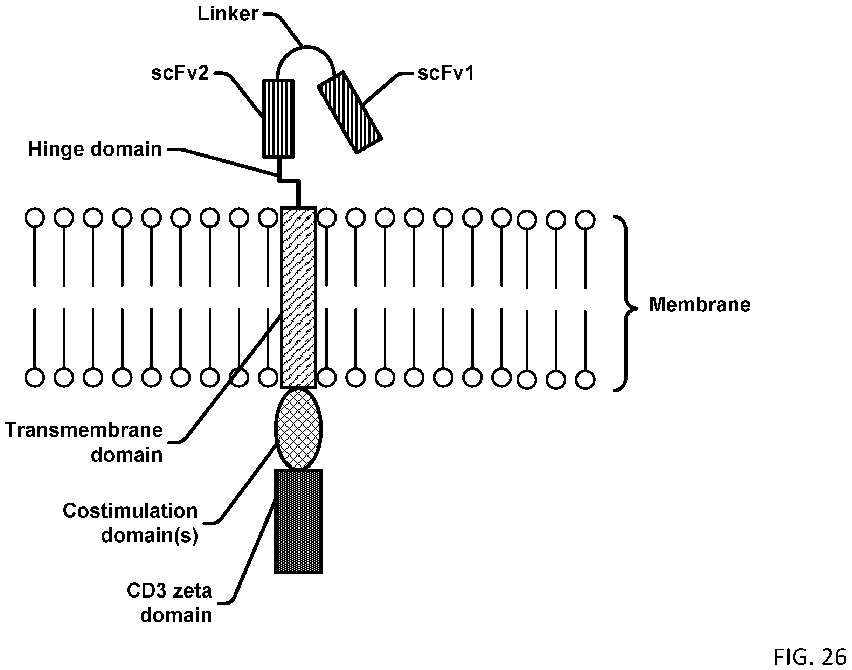

The present disclosure also relates to a bispecific chimeric antigen receptor (See FIG. 26), a polynucleotide encoding the bispecific chimeric antigen receptor, and/or a modified cell comprising the polynucleotide, wherein the bispecific chimeric antigen receptor comprises a first antigen binding domain, a second antigen binding domain, a cytoplasmic domain, and a transmembrane domain, and wherein the first antigen binding domain recognizes a first antigen, and the second antigen binding domain recognize a second antigen. In some disclosures, the first antigen is an antigen associated with a white blood cell, and the second antigen is a solid tumor antigen. In some disclosures, the first and second antigens are identical or different. In some disclosures, the first and second antigens are both solid tumor antigens. For example, in some dislcosures the first antigen is a tumor associated MUC1, and the second antigen is selected from one of the antigens of SEQ ID NO: 1-22. In some disclosures, the first binding domain and the second binding domain are connected via a linker (e.g., SEQ ID NO: 188).

In embodiments, the intracellular domain of the CAR comprises a co-stimulatory signaling region that comprises an intracellular domain of a co-stimulatory molecule selected from the group consisting of CD27, CD28, 4-1BB, OX40, CD30, CD40, PD-1, ICOS, lymphocyte function-associated antigen-1 (LFA-1), CD2, CD7, LIGHT, NKG2C, B7-H3, and any combination thereof.

In embodiments, the intracellular domain comprises a CD3 zeta signaling domain. Embodiments relate to a vector comprising the isolated nucleic acid sequence described herein. Embodiments relate to an isolated cell comprising the isolated nucleic acid sequence described herein.

Embodiments relate to a composition comprising a population of cells including T cells comprising the CAR described herein. Embodiments relate to a CAR encoded by the isolated nucleic acid sequence described herein.

The cells, including CAR cells and modified cells, described herein can be derived from a stem cell. The stem cells may be adult stem cells, embryonic stem cells, or non-human stem cells, cord blood stem cells, progenitor cells, bone marrow stem cells, induced pluripotent stem cells, non-human totipotent stem cells, or hematopoietic stem cells. The cells can also be a dendritic cell, a NK-cell, a B-cell, or a T cell selected from the group consisting of inflammatory T lymphocytes, cytotoxic T lymphocytes, regulatory T lymphocytes, and helper T lymphocytes. In embodiments, the cells can be derived from the group consisting of CD4+T-lymphocytes and CD8+T-lymphocytes. Prior to expansion and genetic modification of the cells described herein, a source of cells may be obtained from a subject through a variety of non-limiting methods. T cells may be obtained from a number of non-limiting sources, including peripheral blood mononuclear cells, bone marrow, lymph node tissue, cord blood, thymus tissue, tissue from a site of infection, ascites, pleural effusion, spleen tissue, and tumors. In embodiments, any number of T cell lines available and known to those skilled in the art, can be used. In embodiments, the cells may be derived from a healthy donor, from a patient diagnosed with cancer or from a patient diagnosed with an infection. In embodiments, the cells are part of a mixed population of cells which present different phenotypic characteristics.

The term "stem cell" refers to any type of cell which has the capacity for self-renewal and the ability to differentiate into other kind(s) of cell. For example, a stem cell gives rise either to two daughter stem cells (as occurs in vitro with embryonic stem cells in culture) or to one stem cell and a cell that undergoes differentiation (as occurs e.g. in hematopoietic stem cells, which give rise to blood cells). Different categories of stem cells may be distinguished on the basis of their origin and/or on the extent of their capacity for differentiation into other types of cell. Stem cells can include embryonic stem (ES) cells (i.e., pluripotent stem cells), somatic stem cells, induced pluripotent stem cells, and any other types stem cells.

Pluripotent embryonic stem cells can be found in the inner cell mass of a blastocyst and have high innate capacity for differentiation. For example, pluripotent embryonic stem cells have the potential to form any type of cell in the body. When grown in vitro for long periods of time, ES cells maintain pluripotency, and progeny cells retain the potential for multilineage differentiation.

Somatic stem cells can include fetal stem cells (from the fetus) and adult stem cells (found in various tissues, such as bone marrow). These cells have been regarded as having a capacity for differentiation lower than that of the pluripotent ES cells - with the capacity of fetal stem cells being greater than that of adult stem cells; they apparently differentiate into only a limited number of different types of cells and have been described as multipotent. "Tissue-specific" stem cells normally give rise to only one type of cell. For example, embryonic stem cells can differentiate into blood stem cells (e.g., Hematopoietic stem cells (HSCs)), which can further differentiate into various blood cells (e.g., red blood cells, platelets, white blood cells, etc.).

Induced pluripotent stem cells (iPS cells or iPSCs) can include a type of pluripotent stem cell artificially derived from a non-pluripotent cell (e.g., an adult somatic cell) by inducing expression of specific genes. Induced pluripotent stem cells are similar to naturally occurring pluripotent stem cells, such as embryonic stem (ES) cells, in many aspects, such as the expression of certain stem cell genes and proteins, chromatin methylation patterns, doubling time, embryoid body formation, teratoma formation, viable chimera formation, and potency and differentiability. Induced pluripotent cells can be isolated from adult stomach, liver, skin cells, and blood cells.

In embodiments, the CAR cells, the modified cell, or the cell is a T cell, a NK cell, a macrophage or a dendritic cell. For example, the CAR cells, the modified cell, or the cell is a T cell.

In some disclosures, the antigen binding molecule is a T Cell Receptor (TCR). In embodiments, the TCR is modified TCR. In embodiments, the TCR is derived from spontaneously occurring tumor-specific T cells in patients. In some disclosures, the TCR binds a tumor antigen. In some disclosures, the tumor antigen comprises CEA, gp100, MART-1, p53, MAGE-A3, or NY-ESO-1. In some disclosures, the TCR comprises TCRγ and TCRδ chains or TCRα and TCRβ chains. In some disclosures, a T cell clone that expresses a TCR with high affinity for the target antigen may be isolated. In embodiments, tumor-infiltrating lymphocytes (TILs) or peripheral blood mononuclear cells (PBMCs) may be cultured in the presence of antigen-presenting cells (APCs) pulsed with a peptide representing an epitope known to elicit a dominant T cell response when presented in the context of a defined HLA allele. High-affinity clones may be then selected on the basis of MHC-peptide tetramer staining and/or the ability to recognize and lyse target cells pulsed with low titrated concentrations of cognate peptide antigen. After the clone has been selected, the TCRα and TCRβ chains or TCRγ and TCRδ chains are identified and isolated by molecular cloning. For example, for TCRα and TCRβ chains, the TCRα and TCRβ gene sequences are then used to generate an expression construct that ideally promotes stable, high-level expression of both TCR chains in human T cells. The transduction vehicle (e.g., a gammaretrovirus or lentivirus) may be then generated and tested for functionality (antigen specificity and functional avidity) and used to produce a clinical lot of the vector. An aliquot of the final product is then used to transduce the target T cell population (generally purified from patient PBMCs), which is expanded before infusion into the subject.

In some disclosures, the binding element of the CAR may include any antigen binding moiety that when bound to its cognate antigen, affects a tumor cell for example, it kills the tumor cell, inhibits the growth of the tumor cell, or promotes death of the tumor cell.

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically, rather than cloned.

The present disclosure further relate to vectors in which a DNA of the present disclosure is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity.

Viruses can be used to deliver nucleic acids into a cell in vitro and in vivo (in a subject). Examples of viruses useful for delivery of nucleic acids into cells include retrovirus, adenovirus, herpes simplex virus, vaccinia virus, and adeno-associated virus.

There also exist non-viral methods for deliverying nucleic acids into a cell, for example, electroporation, gene gun, sonoporation, magnetofection, and the use of oligonucleotides, lipoplexes, dendrimers, and inorganic nanoparticles.

The expression of natural or synthetic nucleic acids encoding CARs is typically achieved by operably linking a nucleic acid encoding the CAR polypeptide or portions thereof to one or more promoters and incorporating the construct into an expression vector. The vectors can be suitable for replication and integration into eukaryotes. Typical cloning vectors contain transcription and translation terminators, initiation sequences, and promoters useful for regulation of the expression of the desired nucleic acid sequence.

Additional information related to expression of synthetic nucleic acids encoding CARs and gene transfer into mammalian cells is provided in U.S. Pat. No. US8,906,682.

Pharmaceutical compositions of the present disclosure may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an immunologically effective amount", "an anti-tumor effective amount", "a tumor-inhibiting effective amount", or "therapeutic amount" is indicated, the precise amount of the compositions of the present disclosure to be administered can be determined by a physician with consideration of individual differences in age, weight, tumor size, extent of infection or metastasis, and condition of the patient (subject). It can be stated that a pharmaceutical composition comprising the T cells described herein may be administered at a dosage of 10⁴ to 10⁹cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988). The optimal dosage and treatment regime for a particular patient can readily be determined by one skilled in the art of medicine by monitoring the patient for signs of disease and adjusting the treatment accordingly. It may be desired to administer activated T cells to a subject and then subsequently redraw the blood (or have apheresis performed), collect the activated and expanded T cells, and reinfuse the patient with these activated and expanded T cells. This process can be carried out multiple times every few weeks. T cells can be activated from blood draws of from 10 cc to 400 cc. T cells are activated from blood draws of 20 cc, 30 cc, 40 cc, 50 cc, 60 cc, 70 cc, 80 cc, 90 cc, or 100 cc. Not to be bound by theory, using this multiple blood draw/multiple reinfusion protocols, certain populations of T cells may be selected.

The administration of the pharmaceutical compositions described herein may be carried out in any convenient manner, including by aerosol inhalation, injection, ingestion, transfusion, implantation or transplantation. The compositions described herein may be administered to a patient subcutaneously, intradermally, intratumorally, intranodally, intramedullary, intramuscularly, by intravenous (i. v.) injection, or intraperitoneally. T cell compositions of the present disclosure are administered to a patient by intradermal or subcutaneous injection. The T cell compositions of the present disclosure are preferably administered by i.v. injection. The compositions of T cells may be injected directly into a tumor, lymph node, or site of infection. Cells may be activated and expanded using the methods described herein, or other methods known in the art where T cells are expanded to therapeutic levels, are administered to a patient in conjunction with (e.g., before, simultaneously or following) any number of relevant treatment modalities, including but not limited to treatment with agents such as antiviral therapy, cidofovir and interleukin-2, Cytarabine (also known as ARA-C) or natalizumab treatment for MS patients or efalizumab treatment for psoriasis patients or other treatments for PML patients. The T cells of the present disclosure may be used in combination with chemotherapy, radiation, immunosuppressive agents, such as cyclosporin, azathioprine, methotrexate, mycophenolate, and FK506, antibodies, or other immunoablative agents such as CAM PATH, anti-CD3 antibodies or other antibody therapies, cytoxin, fludaribine, cyclosporin, FK506, rapamycin, mycophenolic acid, steroids, FR901228, cytokines, and irradiation. These drugs inhibit either the calcium dependent phosphatase calcineurin (cyclosporine and FK506) or inhibit the p70S6 kinase that is important for growth factor induced signaling (rapamycin). (Liu et al., Cell 66:807-815, 1991; Henderson et al., Immun 73:316-321, 1991; Bierer et al., Curr. Opin. Immun 5:763-773, 1993; Isoniemi (supra)). The cell compositions of the present disclosure may be administered to a patient in conjunction with (e.g., before, simultaneously or following) bone marrow transplantation, T cell ablative therapy using either chemotherapy agents such as, fludarabine, external-beam radiation therapy (XRT), cyclophosphamide, or antibodies such as OKT3 or CAMPATH. The cell compositions of the present disclosure may be administered following B-cell ablative therapy such as agents that react with CD20, e.g., Rituxan. For example, subjects may undergo standard treatment with high dose chemotherapy followed by peripheral blood stem cell transplantation. In some disclosures, following the transplant, subjects receive an infusion of the expanded immune cells of the present disclosure. In some disclosures, expanded cells are administered before or following surgery.

The dosage of the above treatments to be administered to a patient will vary with the precise nature of the condition being treated and the recipient of the treatment. The scaling of dosages for human administration can be performed according to art-accepted practices by a physician depending on various factors.

Additional information on the methods of cancer treatment using engineered or modified T cells is provided in U.S. Pat. No. US8,906,682.

The population of cells described herein may be used in autologous CAR T cell therapy. The CAR T cell therapy may be allogenic CAR T cell therapy, TCR T cell therapy, and NK cell therapy.

Disclosed herein is an *in vitro* method for preparing modified cells. The method may include obtaining a sample of cells from the subject. For example, the sample may include T cells or T cell progenitors. The method may further include transfecting the cells with a DNA encoding at least a CAR, culturing the population of CAR cells ex vivo in a medium that selectively enhances proliferation of CAR-expressing T cells.

The sample may be a cryopreserved sample. The sample of cells may be from umbilical cord blood or a peripheral blood sample from the subject. The sample of cells may be obtained by apheresis or venipuncture. The sample of cells may be a subpopulation of T cells.

Also disclosed herein are methods that relate to treating cancer using Chimeric Antigen Receptor (CAR) cells using a molecule associated with a gene fusion. Disclosed methods relate to an isolated nucleic acid sequence encoding a chimeric antigen receptor (CAR), wherein the CAR comprises an extracellular domain, a transmembrane domain, and an intracellular domain, wherein the extracellular domain binds a gene fusion antigen of a gene fusion.

As used herein, the term "gene fusion" refers to the fusion of at least a portion of a gene to at least a portion of an additional gene. The gene fusion need not include entire genes or exons of genes. In some instances, gene fusion is associated with alternations in cancer. A gene fusion product refers to a chimeric genomic DNA, a chimeric messenger RNA, a truncated protein or a chimeric protein resulting from a gene fusion. The gene fusion product may be detected by various methods described in U.S. patent 9938582. A "gene fusion antigen" refers to a truncated protein or a chimeric protein that results from a gene fusion. As disclosed, an epitope of a gene fusion antigen may include a part of the gene fusion antigen or an immunogenic part of another antigen caused by the gene fusion. The gene fusion antigen may interact with, or is part of, cell membranes.

The gene fusion may comprise a fusion of at least a portion of a first gene to at least a portion of a second gene. The first gene and the second gene may comprise a first gene and a second gene of a fusion listed in Table 3. The gene fusion antigen may be associated with a condition listed in the Table 3.

Detection of mRNA and protein expression levels of the target molecules (listed in Table 2) in human cells may be performed using experimental methods such as qPCR and FACS. Further, target molecules specifically expressed in the corresponding tumor cells with very low expression or undetectable expression in normal tissue cells may be identified.

In Vitro Killer Assay as well as killing experiment of CAR T Cells Co-Cultured with Antigen-Positive Cells may be performed. CAR T cells may exhibit a killing effect on the corresponding antigen-positive cells, a decrease in the number of corresponding antigen-positive cells co-cultured with CAR T cells, and an increase in the release of IFNγ, TNFα, etc. as compared to control cells that did not express the corresponding antigen.

In Vivo Killer Assay may be performed. For example, mice may be transplanted with corresponding antigen tumor cells, and tumorigenic, transfusion of CAR T cells, and a decrease in mouse tumors and mouse blood IFNγ, TNFα, and other signals may be defected.

Methods of eliciting and/or enhancing T cell response in a subject having a solid tumor or treating a solid tumor in the subject are also disclosed, the method comprising administering an effective amount of T cells comprising the CAR described herein. The intracellular domain of the CAR may comprise a co-stimulatory signaling region that comprises an intracellular domain of a co-stimulatory molecule selected from the group consisting of CD27, CD28, 4-1BB, OX40, CD30, CD40, PD-1, ICOS, lymphocyte function-associated antigen-1 (LFA-1), CD2, CD7, LIGHT, NKG2C, B7-H3, and any combination thereof. In embodiments, the intracellular domain comprises a CD3 zeta signaling domain.

A vector comprising the isolated nucleic acid described herein is also disclosed.

The invention also provides an isolated cell comprising the isolated nucleic acid sequence described herein. Also disclosed is a composition comprising a population of T cells comprising the CAR described herein. Also disclosed is a CAR encoded by the isolated nucleic acid sequence described herein. Also disclosed is a method of eliciting and/or enhancing T- cell response in a subject or treating a tumor of the subject, the method comprising: administering an effective amount of T cell comprising the CAR described herein.

**Table 3**

| Conditions | Type | Fusion | First gene | Gene description | sublocation | Second Gene | Gene description | sublocation |
|---|---|---|---|---|---|---|---|---|
| breast invasive carcinoma | BRC A | GNASNECTIN2 | GNAS | GNAS complex locus | Plasma membrane | NECTIN2 | Nectin cell adhesion molecule 2 | Plasma membrane |
| breast invasive carcinoma | BRC A | FGFR1-ADAM 18 | FGFR1 | Fibroblast growth factor receptor 1 | Plasma membrane | ADAM 18 | ADAM metallopeptidase domain 18 | Plasma membrane |
| cervical squamous cell carcinoma and endocervical adenocarcinoma | CES C | WHRN--TNC | WHRN | Whirlin | Cytoplasm; Plasma membrane | TNC | Tenascin C | Extracellular ; Plasma membrane |
| head and neck squamous cell carcinoma | HNS C | PQLC1-HSBP1L1 | PQLC1 | PQ loop repeat containing 1 | Plasma membrane | HSBP1L1 | Heat shock factor binding protein 1 like 1 | Plasma membrane |
| kidney renal papillary cell carcinoma | KIRP | FNDC3B-BCHE | FNDC3B | Fibronectin type III domain containing 3B | Plasma membrane | BCHE | Butyrylcholinestera se | Plasma membrane |
| brain lower grade glioma | LGG | GRIA4-NAALAD2 | GRIA4 | Glutamate ionotropic receptor AMPA type subunit 4 | Plasma membrane | NAALAD 2 | N-acetylated alpha-linked acidic dipeptidase 2 | Plasma membrane |
| brain lower grade glioma | LGG | EPHB2-PDZD4 | EPHB2 | EPH receptor B2 | Plasma membrane | PDZD4 | PDZ domain containing 4 | Cytoplasm; Plasma membrane |
| brain lower grade glioma | LGG | SEC24A-KCNK7 | SEC24A | SEC24 homolog A, COPII coat complex component | Plasma membrane | KCNK7 | Potassium two pore domain channel subfamily K member 7 | Plasma membrane |
| liver hepatocellular carcinoma | LIHC | ACVR1B-ACVRL1 | ACVR1B | Activin A receptor type 1B | Plasma membrane | ACVRL1 | Activin A receptor like type 1 | Plasma membrane |
| liver hepatocellular carcinoma | LIHC | ABCC2-CTNNA3 | ABCC2 | ATP binding cassette subfamily C member 2 | Plasma membrane | CTNNA3 | Catenin alpha 3 | Cytoplasm; Cytoskeleto n; Plasma membrane |
| liver hepatocellular carcinoma | LIHC | EFNA1-ADAM 15 | EFNA1 | Ephrin A1 | Extracellula r; Plasma membrane | ADAM 15 | ADAM metallopeptidase domain 15 | Plasma membrane |
| lung adenocarcinoma | LUAD | CPNE8-CADM2 | CPNE8 | Copine 8 | Plasma membrane | CADM2 | Cell adhesion molecule 2 | Plasma membrane |
| lung adenocarcinoma | LUAD | NOTCH2-ADAM30 | NOTCH2 | Notch 2 | Plasma membrane | ADAM30 | ADAM metallopeptidase domain 30 | Plasma membrane |
| lung adenocarcinoma | LUAD | CELSR1-CD52 | CELSR1 | Cadherin EGF LAG seven-pass G-type receptor 1 | Plasma membrane | CD52 | CD52 molecule | Plasma membrane |
| lung adenocarcinoma | LUAD | ILVBL-SLC1A6 | ILVBL | IlvB acetolactate synthase like | Plasma membrane | SLC1A6 | Solute carrier family 1 member 6 | Plasma membrane |
| lung adenocarcinoma | LUAD | F11R-NOS1AP | F11R | F11 receptor | Plasma membrane | NOS1AP | Nitric oxide synthase 1 adaptor protein | Cytoplasm; Plasma membrane |
| lung squamous cell carcinoma | LUSC | CELSR1-SEZ6L | CELSR1 | Cadherin EGF LAG seven-pass G-type receptor 1 | Plasma membrane | SEZ6L | Seizure related 6 homolog like | Plasma membrane |
| lung squamous cell carcinoma | LUSC | KIRREL-CD1A | KIRREL | Kin of IRRE like (Drosophila) | Plasma membrane | CD1A | CD1a molecule | Endosome; Golgi apparatus; Plasma membrane |
| lung squamous cell carcinoma | LUSC | ATP10D-GABRA2 | ATP10D | ATPase phospholipid transporting 10D (putative) | Plasma membrane | GABRA2 | Gamma-aminobutyric acid type A receptor alpha2 subunit | Plasma membrane |
| pancreatic adenocarcinoma | PAA D | ORAI2-SLC47A2 | ORAI2 | ORAI calcium release-activated calcium modulator 2 | Plasma membrane | SLC47A2 | Solute carrier family 47 member 2 | Plasma membrane |
| pheochromocyto ma and paraganglioma | PCP G | ADCYAP1R 1--GHRHR | ADCYAP1R 1 | ADCYAP receptor type I | Plasma membrane | GHRHR | Growth hormone releasing hormone receptor | Plasma membrane |
| pheochromocyto ma and paraganglioma | PCP G | TMEM178B--DPP6 | TMEM178B | Transmembrane protein 178B | Plasma membrane | DPP6 | Dipeptidyl peptidase like 6 | Plasma membrane |
| prostate adenocarcinoma | PRA D | ADAM9-RGS20 | ADAM9 | ADAM metallopeptidase domain 9 | Plasma membrane | RGS20 | Regulator of G-protein signaling 20 | Plasma membrane |
| prostate adenocarcinoma | PRA D | FAM160B1-VTI1A | FAM160B1 | Family with sequence similarity 160 member B1 | Plasma membrane | VTI1A | Vesicle transport through interaction with t-SNAREs 1A | Plasma membrane |
| prostate adenocarcinoma | PRA D | TMPRSS2-PDE9A | TM PRSS2 | Transmembrane protease, serine 2 | Plasma membrane | PDE9A | Phosphodiesterase 9A | Plasma membrane |
| prostate adenocarcinoma | PRA D | PDE9A-TMPRSS2 | PDE9A | Phosphodiestera se 9A | Plasma membrane | TMPRSS 2 | Transmembrane protease, serine 2 | Plasma membrane |
| rectum adenocarcinoma | REA D | LHFPL2-PTPRK | LHFPL2 | Lipoma HMGIC fusion partner-like 2 | Plasma membrane | PTPRK | Protein tyrosine phosphatase, receptor type K | Plasma membrane |
| sarcoma | SAR C | TM7SF3-KCNC2 | TM7SF3 | Transmembrane 7 superfamily member 3 | Plasma membrane | KCNC2 | Potassium voltage-gated channel subfamily C member 2 | Plasma membrane |
| sarcoma | SAR C | MPZL1-TNFSF4 | MPZL1 | Myelin protein zero like 1 | Plasma membrane | TNFSF4 | Tumor necrosis factor superfamily member 4 | Plasma membrane |
| sarcoma | SAR C | GNG7-PAQR5 | GNG7 | G protein subunit gamma 7 | Plasma membrane | PAQR5 | Progestin and adipoQ receptor family member 5 | Plasma membrane |
| sarcoma | SAR C | KIRREL-CD1A | KIRREL | Kin of IRRE like (Drosophila) | Plasma membrane | CD1A | CD1a molecule | Endosome; Golgi apparatus; Plasma membrane |
| sarcoma | SAR C | P2RX5-TRPV1 | P2RX5 | Purinergic receptor P2X 5 | Plasma membrane | TRPV1 | Transient receptor potential cation channel subfamily V member 1 | Plasma membrane |
| skin cutaneous melanoma | SKC M | PTPRGSYNPR | PTPRG | Protein tyrosine phosphatase, receptor type G | Plasma membrane | SYNPR | Synaptoporin | Plasma membrane |

The CAR molecules of the invention described herein comprises: a heavy chain variable region (HVR) comprising amino acid sequence SEQ ID NO: 83 and a light chain variable region (LVR) comprising amino acid sequence SEQ ID NO: 82. In other disclosures, the CAR molecules comprise one or more complementarity-determining regions (CDRs) for binding an antigen of interest. CDRs are part of the variable domains in immunoglobulins and T cell receptors for binding a specific antigen. There are three CDRs for each variable domain. Since there is a variable heavy domain and a variable light domain, there are six CDRs for binding an antigen. Further since an antibody has two heavy chains and two light chains, an antibody has twelve CDRs altogether for binding antigens. In some disclosures, the CAR molecules comprise one or more CDRs of SIGLEC15, SLC6A3, KISS1R, QRFPR, GPR119, CLDN6, UPK2, ADAM12, SLC45A3, ACPP, MUC21, MUC16, MS4A12, or ALPP.

The present disclosure describes modified cells that include one or more different antigen binding domains. The modified cells can include at least two different antigen binding domains: a first antigen binding domain for expanding and/or maintaining the genetically modified cells, and a second antigen binding domain for killing a target cell, such as a tumor cell. In the invention at least one of the antigen binding domains comprises: a heavy chain variable region (HVR) comprising amino acid sequence SEQ ID NO: 83 and a light chain variable region (LVR) comprising amino acid sequence SEQ ID NO: 82. In other disclosures for example, the first antigen binding domain binds a surface marker, such as a cell surface molecule of a white blood cell (WBC) (e.g., CD19), and the second antigen binding domain binds a target antigen on tumor cells. In some disclosures, the cell surface molecule is a surface antigen of a WBC. In some disclosures, the target antigen on tumor cells comprise one or more of SIGLEC15, SLC6A3, KISS1R, QRFPR, GPR119, CLDN6, UPK2, ADAM12, SLC45A3, ACPP, MUC21, MUC16, MS4A12, or ALPP. The at least two antigen binding domains may be located on the same or different modified cells. For example, the modified cells may include a modified cell including a CAR binding CD19, a modified cell including a CAR binding to ACPP, a modified cell including a CAR binding CD19 and ACPP, and/or a modified cell including two CARs that respectively bind CD19 and ACPP. In some disclosures, the modified cells may be used to treat a subject having cancer.

In the invention, in some instances, the modified cells described herein includes a CAR molecule comprising at least two different antigen binding domains, wherein one of the antigen binding domains comprises: a heavy chain variable region (HVR) comprising amino acid sequence SEQ ID. The CAR molecule can be a bispecific CAR molecule. For example, the two antigen binding domains can be on the same CAR molecule, on different CAR molecules, or on a CAR molecule and T cell receptor (TCR). A single CAR can include at least two different antigen binding domains, or the two different antigen binding domains are each on a separate CAR molecule. The at least two different antigen binding domains can be on the same CAR molecule or different CAR molecules, but in the same modified cell. Moreover, the at least two different antigen binding domains can be on a CAR molecule and a T cell receptor in the same modified cell. In some disclosures, the bispecific CAR molecule may include a binding domain binding an antigen of WBC (e.g., CD19) and a binding domain binding a solid tumor antigen. In embodiments, the bispecific CAR molecule may include two binding domains binding two different solid tumor antigens.

In embodiments, the at least two different antigen binding domains are on different CAR molecules which are expressed by different modified cells. Further, the one or more different antigen binding domains are on a CAR molecule and a T cell receptor, which are expressed by different modified cells.

Related sequences are provided in this Application and Innovative Cellular Therapeutics' PCT patent application PCT/CN2016/075061.

### EXAMPLES

The present disclosure is further described by reference to the following examples. These examples are provided for purposes of illustration only and are not intended to be limiting unless otherwise specified.

### Expression of CAR on T cells

Lentiviral vectors that encode a CAR were generated (see Chimeric Receptors Containing CD137 Signal Transduction Domains Mediate Enhanced Survival of T Cells and Increased Antileukemic Efficacy In Vivo Molecular Therapy vol. 17 no. 8, 1453-1464 Aug. 2009) and were introduced into human T cells.

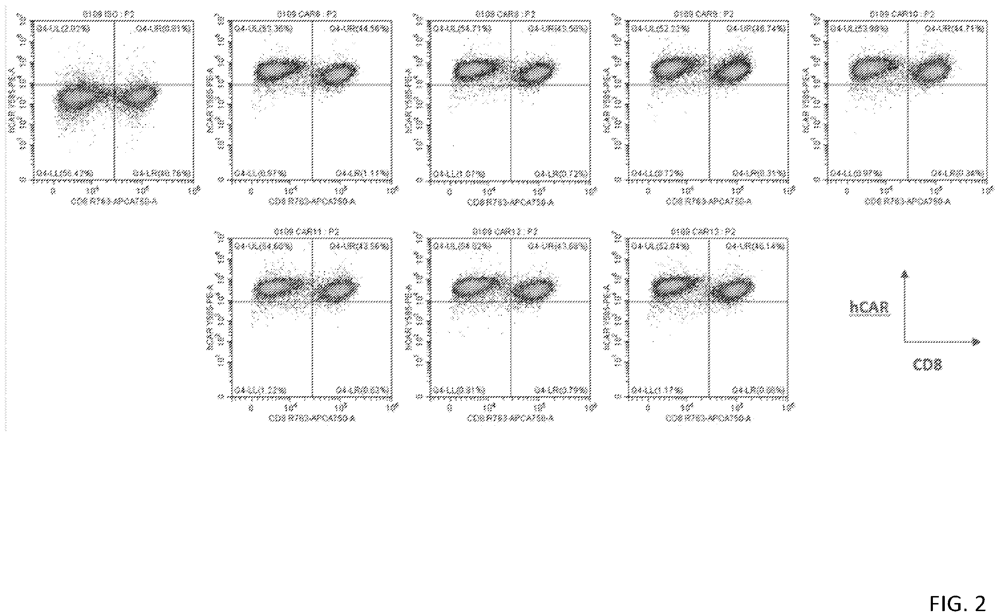

Primary T cells were obtained from patients. The obtained primary T cells were transduced with lentiviral vectors to obtain modified T cells. Flow-cytometry was performed and analyzed to confirm the expression of CARs in primary T cells (FIG. 2). Techniques related to cell cultures, construction of lentiviral vectors, and flow cytometry may be found in Control of large, established tumor xenografts with genetically retargeted human T cells containing CD28 and CD137 domains. 3360-3365 PNAS March 3, 2009, vol. 106 no. 9.

### Expression of antigens and related tumor analysis

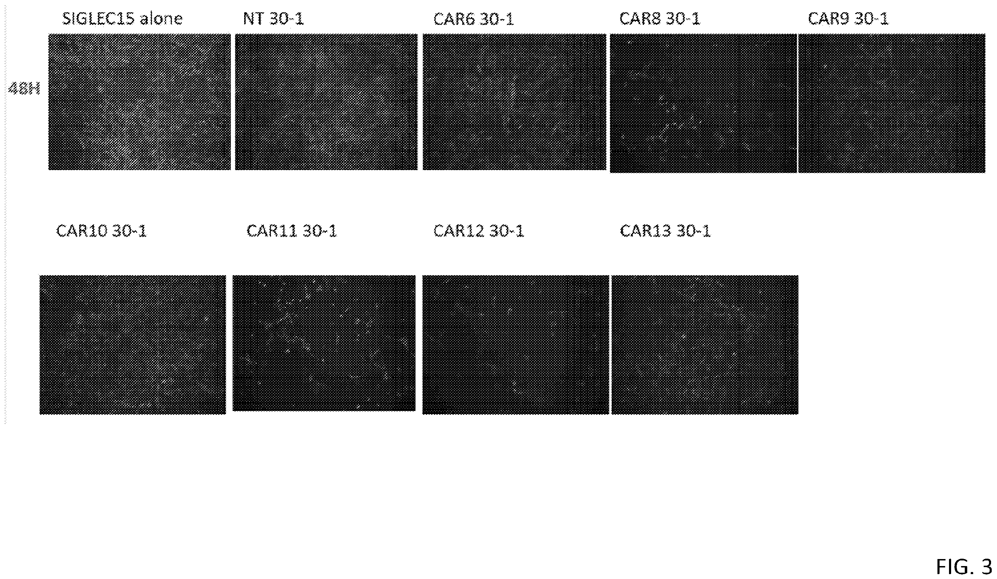

Detection of mRNA and protein expression levels of target molecules in human cells using experimental methods such as qPCR and FACS were performed. It has been shown that some of the target molecules listed below are specifically expressed in the corresponding tumor cells with relatively low expression or undetectable expression in normal tissue. For example, eight genes were localized in normal tissues and overexpressed in tumor cells. The target point and the corresponding relationship between the tumor are provided in the Table 4. Various assays (e.g., killing) were perform, and results are shown in FIGS. 3-7. Techniques related to cell cultures, construction of cytotoxic T-lymphocyte assay may be found in "Control of large, established tumor xenografts with genetically retargeted human T cells containing CD28 and CD137 domains," PNAS, March 3, 2009, vol. 106 no. 9, 3360-3365.

**Table 4**

| Gene name | Subcellular localization | Organ mainly expressing | Target Tumor | Target SEQ ID NO. |
|---|---|---|---|---|
| *SIGLEC15* | Plasma membrane | Expression in all normal tissues but very low | Urothelial cancer | 17 |
| *SLC6A3* | Plasma membrane | Expression in all normal tissues but very low | Renal cancer | 18 |
| *KISS1R* | Plasma membrane | Expression in all normal tissues but very low | Renal cancer | 19 |
| *QRFPR* | Plasma membrane | Expression in all normal tissues but very low | Renal cancer: | 20 |
| *GPR119* | Plasma membrane | Expression in all normal tissues but very low | Pancreatic cancer | 21 |
| *CLDN6* | Plasma membrane | Expression in all normal tissues but very low | Endometrial cancer/ Urothelial cancer | 22 |
| *UPK2* | Plasma membrane | Urethra / bladder | Urothelial cancer (including bladder cancer) | 1 |
| *ADAM12* | Plasma membrane | placenta | Breast cancer, pancreatic cancer and the like | 2 |
| *SLC45A3* | Plasma membrane | prostate | Prostate cancer | 3 |
| *ACPP* | Plasma membrane | prostate | Prostate cancer | 4 |
| *MUC21* | Plasma membrane | esophagus | Esophageal cancer | 5 |
| *MUC16* | Plasma membrane | Cervical / Fallopian tube | Ovarian cancer | 6 |
| *MS4A12* | Plasma membrane | the large intestine | Colorectal cancer | 7 |
| *ALPP* | Plasma membrane | Placenta / cervix | Endometrial cancer | 8 |

### Identifying gene fusion antigens

Databases (e.g., TCGA) were analyzed to identify gene fusion products and gene fusion antigens. Hundreds of gene fusion products were identified and determined to be associated with cancer. Among these hundreds of gene fusion products, 33 gene fusion antigen groups were further identified based on predetermined criteria. For example, these gene fusion products were found to be expressed in tumor cells, and both fusion proteins of the fusion genes were located in the cytoplasmic membrane. Information of these 33 gene fusion antigen groups are listed in Table 5. Samples corresponding to each row of Table 5 include TCGA-D8-A1XJ-01A-11R-A14M-07, TCGA-E2-A574-01A-11R-A29R-07, TCGA-FU-A3TX-01A-11R-A22U-07, TCGA-CV-7434-01A-11R-2132-07, TCGA-G7-6793-01A-11R-1965-07, TCGA-DB-5277-01A-01R-1470-07, TCGA-KT-A7W1-01A-11R-A34F-07, TCGA-VM-A8CD-01A-11R-A36H-07, TCGA-DD-A3A5-01A-11R-A22L-07, TCGA-DD-AAEA-01A-11R-A41C-07, TCGA-ZS-A9CF-01A-11R-A38B-07, TCGA-44-7670-01A-11R-2066-07, TCGA-55-7281-01A-11R-2039-07, TCGA-62-8394-01A-11R-2326-07, TCGA-78-7143-01A-11R-2039-07, TCGA-78-7146-01A-11R-2039-07, TCGA-46-6025-01A-11R-1820-07, TCGA-85-7710-01A-11R-2125-07, TCGA-02-A52V-01A-31R-A262-07, TCGA-FB-AAPZ-01A-11R-A41B-07, TCGA-P8-A5KC-01A-11R-A35K-07, TCGA-WB-A81D-01A-11R-A35L-07, TCGA-EJ-5510-01A-01R-1580-07, TCGA-EJ-A46G-01A-31R-A26U-07, TCGA-ZG-A9L2-01A-31R-A41O-07, TCGA-ZG-A9L2-01A-31R-A41O-07, TCGA-EI-7002-01A-11R-1928-07, TCGA-DX-A1KZ-01A-11R-A24X-07, TCGA-DX-A2J0-01A-11R-A21T-07, TCGA-DX-A3UF-01A-11R-A30C-07, TCGA-DX-AB2V-01A-11R-A41I-07, TCGA-K1-A6RU-01A-11R-A32Q-07, and TCGA-EB-A24D-01A-11R-A18T-07, each representing a barcode in TCGA database. TCGA barcodes, represents the metadata of the participants and their samples in TCGA database.

**Table 5**

| **Cancer** | **Cancer _type** | **Fusion** | **Junct ion** | **Span ning** | **Breakpoint 1** | **Breakpoint 2** | **gene1** | **Gene description** | **subloc ation** | **gene2** | **Gene description2** | **subloca tion3** |
|---|---|---|---|---|---|---|---|---|---|---|---|---|
| breast invasive carcinoma | BRCA | GNASNECTIN 2 | 23 | 51 | chr20:58895 684:+ | chr19:44882 211:+ | GNAS | GNAS complex locus | Plasma membra ne | NECTI N2 | Nectin cell adhesion molecule 2 | Plasma membra ne |
| breast invasive carcinoma | BRCA | FGFR1-ADAM 18 | 9 | 42 | chr8:384573 56:- | chr8:396063 07:+ | FGFR1 | Fibroblast growth factor receptor 1 | Plasma membra ne | ADAM 18 | ADAM metallopeptid ase domain 18 | Plasma membra ne |
| cervical squamous cell carcinoma and endocervical adenocarcin oma | CESC | WHRN-TNC | 1000 | 1000 | chr9:114403 306:- | chr9:115035 229:- | WHRN | Whirlin | Cytopla sm; Plasma membra ne | TNC | Tenascin C | Extracell ular; Plasma membra ne |
| head and neck squamous cell carcinoma | HNSC | PQLC1-HSBP1L 1 | 28 | 210 | chr18:79943 329:- | chr18:79966 612:+ | PQLC1 | PQ loop repeat containing 1 | Plasma membra ne | HSBP 1L1 | Heat shock factor binding protein 1 like 1 | Plasma membra ne |
| kidney renal papillary cell carcinoma | KIRP | FNDC3B --BCHE | 91 | 307 | chr3:172133 546:+ | chr3:165786 311:- | FNDC3 B | Fibronectin type III domain containing 3B | Plasma membra ne | BCHE | Butyrylcholin esterase | Plasma membra ne |
| brain lower grade glioma | LGG | GRIA4-NAALAD 2 | 28 | 113 | chr11:10586 2208:+ | chr11:90173 824:+ | GRIA4 | Glutamate ionotropic receptor AMPA type subunit 4 | Plasma membra ne | NAAL AD2 | N-acetylated alpha-linked acidic dipeptidase 2 | Plasma membra ne |
| brain lower grade glioma | LGG | EPHB2-PDZD4 | 6 | 29 | chr1:227850 76:+ | chrX:15380 8595:- | EPHB2 | EPH receptor B2 | Plasma membra ne | PDZD 4 | PDZ domain containing 4 | Cytoplas m; Plasma membra ne |
| brain lower grade glioma | LGG | SEC24A --KCNK7 | 11 | 140 | chr5:134649 173:+ | chr11:65593 874:- | SEC24A | SEC24 homolog A, COPII coat complex component | Plasma membra ne | KCNK 7 | Potassium two pore domain channel subfamily K member 7 | Plasma membra ne |
| liver hepatocellul ar carcinoma | LIHC | ACVR1B ACVRL1 | 6 | 43 | chr12:51951 834:+ | chr12:51912 470:+ | ACVR1 B | Activin A receptor type 1B | Plasma membra ne | ACVR L1 | Activin A receptor like type 1 | Plasma membra ne |
| liver hepatocellul ar carcinoma | LIHC | ABCC2-CTNNA3 | 192 | 315 | chr10:99784 781:+ | chr10:65920 617:- | ABCC2 | ATP binding cassette subfamily C member 2 | Plasma membra ne | CTNN A3 | Catenin alpha 3 | Cytoplas m; Cytoskel eton; Plasma membra ne |
| liver hepatocellul ar carcinoma | LIHC | EFNA1-ADAM 15 | 5 | 17 | chr1:155131 634:+ | chr1:155062 460:+ | EFNA1 | Ephrin A1 | Extracel lular; Plasma membra ne | ADAM 15 | ADAM metallopeptid ase domain 15 | Plasma membra ne |
| lung adenocarcin oma | LUAD | CPNE8-CADM2 | 16 | 74 | chr12:38905 437:- | chr3:858020 47:+ | CPNE8 | Copine 8 | Plasma membra ne | CADM 2 | Cell adhesion molecule 2 | Plasma membra ne |
| lung adenocarcin oma | LUAD | NOTCH 2-ADAM30 | 1000 | 1000 | chr1:119935 472:- | chr1:119894 596:- | NOTCH 2 | Notch 2 | Plasma membra ne | ADAM 30 | ADAM metallopeptid ase domain 30 | Plasma membra ne |
| lung adenocarcin oma | LUAD | CELSR1 --CD52 | 81 | 308 | chr22:46463 707:- | chr1:263201 71:+ | CELSR 1 | Cadherin EGF LAG seven-pass G-type receptor 1 | Plasma membra ne | CD52 | CD52 molecule | Plasma membra ne |
| lung adenocarcin oma | LUAD | ILVBL-SLC1A6 | 27 | 67 | chr19:15122 693:- | chr19:14950 390:- | ILVBL | IIvB acetolactate synthase like | Plasma membra ne | SLC1 A6 | Solute carrier family 1 member 6 | Plasma membra ne |
| lung adenocarcin oma | LUAD | F11R-NOS1A P | 29 | 220 | chr1:161021 010:- | chr1:162355 187:+ | F11R | F11 receptor | Plasma membra ne | NOS1 AP | Nitric oxide synthase 1 adaptor protein | Cytoplas m; Plasma membra ne |
| lung squamous cell carcinoma | LUSC | CELSR1 --SEZ6L | 137 | 521 | chr22:46533 627:- | chr22:26292 406:+ | CELSR 1 | Cadherin EGF LAG seven-pass G-type receptor 1 | Plasma membra ne | SEZ6 L | Seizure related 6 homolog like | Plasma membra ne |
| lung squamous cell carcinoma | LUSC | KIRREL- -CD1A | 10 | 64 | chr1:157993 728:+ | chr1:158255 084:+ | KIRREL | Kin of IRRE like (Drosophila) | Plasma membra ne | CD1A | CD1a molecule | Endoso me; Golgi apparatu s; Plasma membra ne |
| lung squamous cell carcinoma | LUSC | ATP10D GABRA 2 | 20 | 79 | chr4:475256 42:+ | chr4:462506 04:- | ATP10D | ATPase phospholipi d transporting 10D (putative) | Plasma membra ne | GABR A2 | Gamma-aminobutyric acid type A receptor alpha2 subunit | Plasma membra ne |
| pancreatic adenocarcin oma | PAAD | ORAI2-SLC47A 2 | 7 | 17 | chr7:102439 181:+ | chr17:19681 670:- | ORAI2 | ORAI calcium release-activated calcium modulator 2 | Plasma membra ne | SLC4 7A2 | Solute carrier family 47 member 2 | Plasma membra ne |
| pheochromo cytoma and paraganglio ma | PCPG | ADCYA P1R1-GHRHR | 5 | 25 | chr7:311002 10:+ | chr7:309749 71:+ | ADCYA P1R1 | ADCYAP receptor type I | Plasma membra ne | GHRH R | Growth hormone releasing hormone receptor | Plasma membra ne |
| pheochromo cytoma and paraganglio ma | PCPG | TMEM1 78B-DPP6 | 15 | 107 | chr7:141212 704:+ | chr7:154769 417:+ | TMEM1 78B | Transmemb rane protein 178B | Plasma membra ne | DPP6 | Dipeptidyl peptidase like 6 | Plasma membra ne |
| prostate adenocarcin oma | PRAD | ADAM9- -RGS20 | 5 | 28 | chr8:389971 60:+ | chr8:538792 58:+ | ADAM9 | ADAM metallopepti dase domain 9 | Plasma membra ne | RGS2 0 | Regulator of G-protein signaling 20 | Plasma membra ne |
| prostate adenocarcin oma | PRAD | FAM160 B1-VTI1A | 10 | 35 | chr10:11483 6246:+ | chr10:11266 8218:+ | FAM 160 B1 | Family with sequence similarity 160 member B1 | Plasma membra ne | VTI1A | Vesicle transport through interaction with t-SNAREs 1A | Plasma membra ne |
| prostate adenocarcin oma | PRAD | TMPRS S2-PDE9A | 22 | 56 | chr21:41494 356:- | chr21:42743 776:+ | TMPRS S2 | Transmemb rane protease, serine 2 | Plasma membra ne | PDE9 A | Phosphodiest erase 9A | Plasma membra ne |
| prostate adenocarcin oma | PRAD | PDE9A-TMPRS S2 | 17 | 40 | chr21:42733 426:+ | chr21:41498 189:- | PDE9A | Phosphodie sterase 9A | Plasma membra ne | TMPR SS2 | Transmembr ane protease, serine 2 | Plasma membra ne |
| rectum adenocarcin oma | READ | LHFPL2- -PTPRK | 8 | 62 | chr5:785097 84:- | chr6:128089 992:- | LHFPL2 | Lipoma HMGIC fusion partner-like 2 | Plasma membra ne | PTPR K | Protein tyrosine phosphatase, receptor type K | Plasma membra ne |
| sarcoma | SARC | TM7SF3 KCNC2 | 13 | 82 | chr12:26990 450:- | chr12:75051 317:- | TM7SF3 | Transmemb rane 7 superfamily member 3 | Plasma membra ne | KCNC 2 | Potassium voltage-gated channel subfamily C member 2 | Plasma membra ne |
| sarcoma | SARC | MPZL1-TNFSF4 | 16 | 175 | chr1:167776 166:+ | chr1:173188 569:- | MPZL1 | Myelin protein zero like 1 | Plasma membra ne | TNFS F4 | Tumor necrosis factor superfamily member 4 | Plasma membra ne |
| sarcoma | SARC | GNG7-PAQR5 | 5 | 6 | chr19:25206 08:- | chr15:69399 974:+ | GNG7 | G protein subunit gamma 7 | Plasma membra ne | PAQR 5 | Progestin and adipoQ receptor family member 5 | Plasma membra ne |
| sarcoma | SARC | KIRREL- -CD1A | 1000 | 1000 | chr1:157993 728:+ | chr1:158256 786:+ | KIRREL | Kin of IRRE like (Drosophila) | Plasma membra ne | CD1A | CD1a molecule | Endoso me; Golgi apparatu s; Plasma membra ne |
| sarcoma | SARC | P2RX5-TRPV1 | 28 | 103 | chr17:36795 90:- | chr17:35722 49:- | P2RX5 | Purinergic receptor P2X 5 | Plasma membra ne | TRPV 1 | Transient receptor potential cation channel subfamily V member 1 | Plasma membra ne |
| skin cutaneous melanoma | SKCM | PTPRG--SYNPR | 6 | 55 | chr3:617489 82:+ | chr3:634808 32:+ | PTPRG | Protein tyrosine phosphatas e, receptor type G | Plasma membra ne | SYNP R | Synaptoporin | Plasma membra ne |

**Table 6: Identifier and related Sequences**

| Identifier | SEQ ID NO: | Identifier | SEQ ID NO: | Identifier | SEQ ID NO: |
|---|---|---|---|---|---|
| UPK2 | 1 | Hinge | 25 | VL1 VH1 SIGLEC-15-CAR-2 | 49 |
| ADAM 12 | 2 | TM | 26 | VL1 VH2 SIGLEC-15-CAR-3 | 50 |
| SLC45A3 | 3 | 4-1BB | 27 | VL1 VH3 SIGLEC-15-CAR-4 | 51 |
| ACPP | 4 | CD3 zeta | 28 | VL1 VH 4 SIGLEC-15-CAR-5 | 52 |
| MUC21 | 5 | CLDN6-CAR-1 | 29 | VL2 VH 1 SIGLEC-15-CAR-6 | 53 |
| MUC16 | 6 | ScFv CLDN6-CAR-1 | 30 | VL2 VH2 SIGLEC-15-CAR-7 | 54 |
| MS4A12 | 7 | ScFv VL CLDN6-CAR-1 | 31 | VL2 VH3 SIGLEC-15-CAR-8 | 55 |
| ALPP | 8 | ScFv VH CLDN6-CAR-1 | 32 | VL2 VH4 SIGLEC-15-CAR-9 | 56 |
| SLC2A14 | 9 | CLDN6-CAR-2 | 33 | VL1 SIGLEC-15-CAR | 57 |
| GS1-259H13.2 | 10 | ScFv CLDN6-CAR-2 | 34 | VL2 SIGLEC-15-CAR | 58 |
| ERVFRD-1 | 11 | ScFv VL CLDN6-CAR-2 | 35 | VH1 SIGLEC-15-CAR | 59 |
| ADGRG2 | 12 | ScFv VH CLDN6-CAR-2 | 36 | VH2 SIGLEC-15-CAR | 60 |
| ECEL1 | 13 | CLDN6-CAR-3 | 37 | VH3 SIGLEC-15-CAR | 61 |
| CHRNA2 | 14 | scFv CLDN6-CAR-3 | 38 | VH4 SIGLEC-15-CAR | 62 |
| GP2 | 15 | scFv VL CLDN6-CAR-3 | 39 | MUC16-CAR-1 | 63 |
| PSG9 | 16 | scFv VH CLDN6-CAR-3 | 40 | scFv MUC16-CAR-1 | 64 |
| SIGLEC15 | 17 | CLDN6-CAR-4 | 41 | scFv VL MUC16-CAR-1 | 65 |
| SLC6A3 | 18 | scFv CLDN6-CAR-4 | 42 | scFv VH MUC16-CAR-1 | 66 |
| KISS1R | 19 | scFv VL CLDN6-CAR-4 | 43 | MUC16-CAR-2 | 67 |
| QRFPR | 20 | scFv VH CLDN6-CAR-4 | 44 | scFv MUC16-CAR-2 | 68 |
| GPR119 | 21 | SIGLEC-15-CAR-1 | 45 | scFv VL MUC16-CAR-2 | 69 |
| CLDN6 | 22 | scFv SIGLEC-15-CAR-1 | 46 | scFv VH MUC16-CAR-2 | 70 |
| SP | 23 | scFv VL SIGLEC-15-CAR-1 | 47 | KISS1R-CAR | 71 |
| Linker | 24 | scFv VH SIGLEC-15-CAR-1 | 48 | Ligent peptide KISS1R-CAR | 72 |
| 6503 S5D1 VL NA | 73 | 6503 S5D1 VL aa | 82 | scFv UPK2-S3D10 NA | 107 |
| 6503 S5D1 VH NA | 74 | 6503 S5D1 VH aa | 83 | scFv UPK2-S3D10 aa | 108 |
| 6504 S5F2 VL NA | 75 | 6504 S5F2 VL aa | 84 | VL UPK2-S3D10 aa | 109 |
| 6504 S5F2 VH NA | 76 | 6504 S5F2 VH aa | 85 | VH UPK2-S3D10 aa | 110 |
| 6502 S12E9 VL NA | 77 | 6502 S12E9 VL aa | 86 | scFv UPK2-S7F9 NA | 111 |
| 6502 S12E9 VH NA | 78 | 6502 S12E9 VH aa | 87 | scFv UPK2-S7F9 aa | 112 |
| 6501 S10E12 VL NA | 79 | 6501 S10E12 VL aa | 88 | VL UPK2-S7F9 aa | 113 |
| 6501 S10E12 VH NA | 80 | 6501 S10E12 VH aa | 89 | VH UPK2-S7F9 aa | 114 |
| 3xGGGGS NA | 81 | 3xGGGGS aa | 90 | scFv UPK2-S7F11 NA | 115 |
| scFv UPK2-S2B7 NA | 91 | scFv UPK2-S3A4 NA | 99 | scFv UPK2-S7F11 aa | 116 |
| scFv UPK2-S2B7 aa | 92 | scFv UPK2-S3A4 AA | 100 | VL UPK2-S7F11 aa | 117 |
| VH UPK2-S2B7 aa | 93 | VL UPK2-S3A4 AA | 101 | VH UPK2-S7F11 aa | 118 |
| VL UPK2-S2B7 aa | 94 | VH UPK2-S3A4 AA | 102 | scFv UPK2-S7F11 NA | 119 |
| scFv UPK2-S2E2 NA | 95 | scFv UPK2-S3C10 NA | 103 | scFv UPK2-S7F11 aa | 120 |
| scFv UPK2-S2E2 aa | 96 | scFv UPK2-S3C10 AA | 104 | VL UPK2-S7F11 aa | 121 |
| VL UPK2-S2E2 aa | 97 | VL UPK2-S3C10 AA | 105 | VH UPK2-S7F11 aa | 122 |
| VH UPK2-S2E2 aa | 98 | VH UPK2-S3C10 AA | 106 | MUC1-1 HL CD3 HL | 123 |
| MUC1-2 HL CD3 HL | 124 | FZD10 HL CD3 HL | 125 | TSHR HL CD3 HL | 126 |
| PRLR HL CD3 HL | 127 | MUC17 HL CD3 HL | 128 | GUCY2C HL CD3 HL | 129 |
| CD207 HL CD3 HL | 130 | CLDN18.2 HL CD3 HL | 131 | CLDN6 HL CD3HL | 132 |
| SIGL1C-15 HL CD3 HL | 133 | muc16 HL cd3 HL | 134 | Cd3 HL | 135 |
| scFv TSHR | 136 | scFv PRLR | 137 | scFv Muc 17 | 138 |
| scFv GUCY2C | 139 | scFv CD207 | 140 | Prolactin (ligand) | 141 |
| scFv CD3 | 142 | scFv CD4 | 143 | scFv CD4-2 | 144 |
| scFv CD5 | 145 | scfv CD33 | 146 | scfv CD123 | 147 |
| scfv ROR1 | 148 | scfv CD70 | 149 | scfv CD133 | 150 |
| scfv GPC3 | 151 | scfv EpCAM | 152 | scfv CD20 | 153 |
| scfv CD22 | 154 | scfv CD30 | 155 | scfv CD5 | 156 |
| scfv Her2 | 157 | scfv CEA | 158 | scfv PSCA | 159 |
| scfv TAG-72 | 160 | scfv CD38 | 161 | scfv EGFRV III | 162 |
| scfv EphA2 | 163 | scfv FAP | 164 | scfv GD2 | 165 |
| scfv GD3 | 166 | scfv IL13R-2a | 167 | scfv NKG2D | 168 |
| scfv PSMA | 169 | scfv survivin | 170 | Tumor associated MUC1 scFv | 171 |
| scfv Mesothelin | 172 | scFv 6503 S5D1 | 173 | scFv 6504 S5F2 | 174 |
| scFv 6502 S12E9 | 175 | scFv 6501 S10E12 | 176 | scFv S4C7 | 177 |
| scFv S12D4 | 178 | scFv S9F5 | 179 | 6502 S12E9 VL | 180 |
| 6502 S12E9 VH | 181 | S4C7VH | 182 | S4C7VL | 183 |
| S12D4VH | 184 | S12D4VL | 185 | S9F5VH | 186 |
| S9F5VL | 187 | 4xGGGGS | 188 | PmCGR | 189 |
| PmCKR | 190 | | | | |
| scFv: Signal peptide + V L + 3xGGGGS + VH | | | | | |

### Anti-SINGLEC-15 CAR and Anti-KISSR CAR

FIG. 2 shows flow cytometry analysis of T cells expressing CARs. On Day 1, CD3 positive T cells were sorted. On Day 3, the T cells were infected with lentivirus encoding CAR (multiplicity of infection (MOI): 50:1), On Day 5-6, expression assay was performed. On Day 6, T cells were cultured with 3T3-antigen-mcherry-SC with ratio 30:1. Sequences and corresponding references listed in FIGS. 2-7 are listed in Table 7.

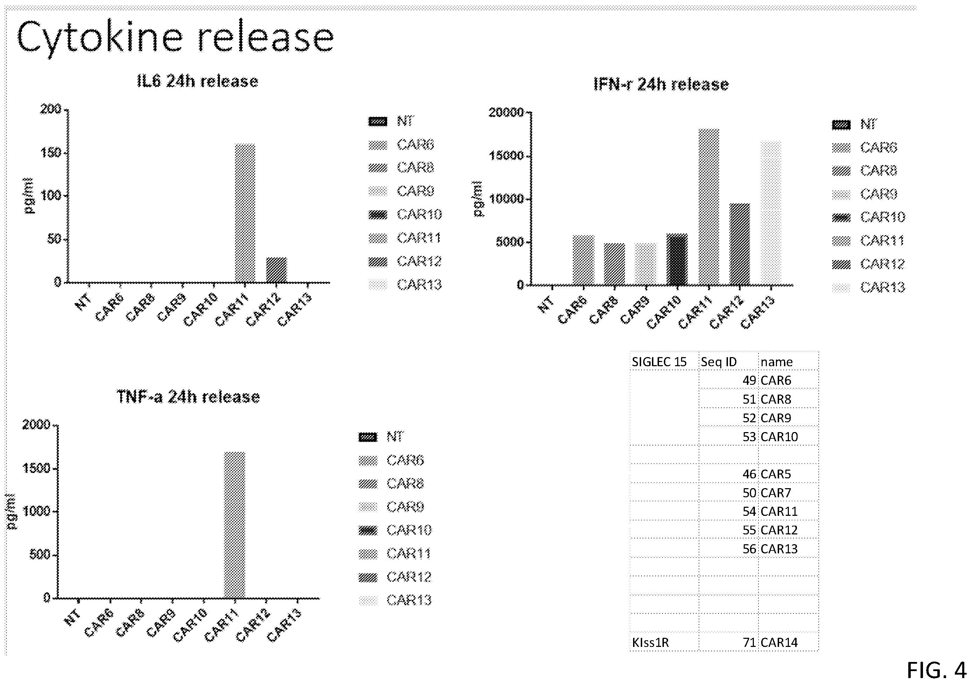

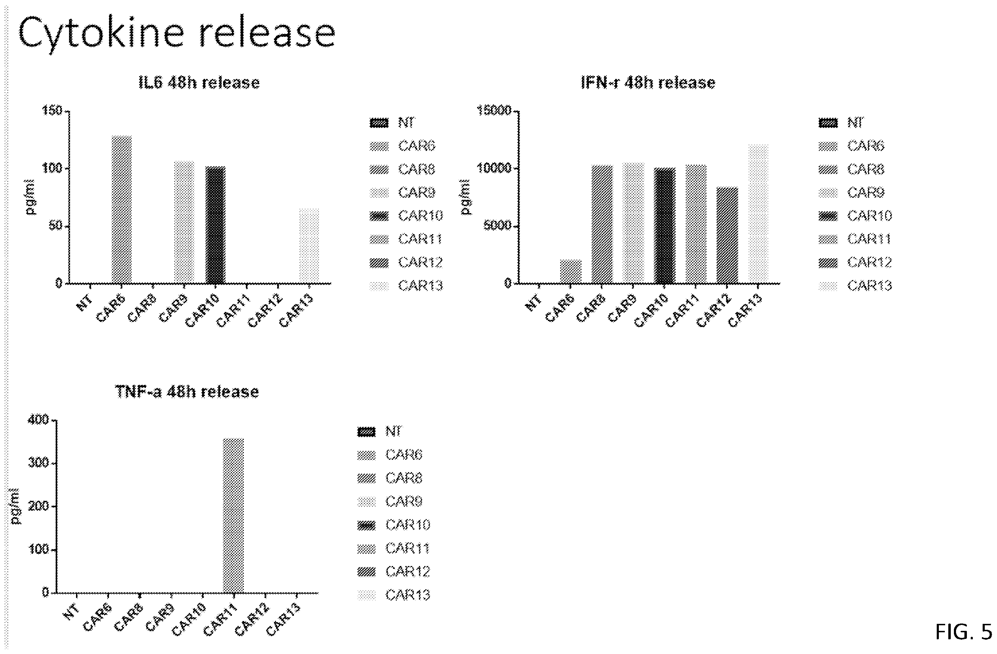

FIG. 3 shows results of a killing assay of anti-SIGLEC15 CAR (SIGLEC15-CAR SEQ ID NO: 46, 50, 51, and 52). It was observed that anti-SIGLEC15 CAR T cells killed the substrate cells. The negative control SIGLEC15 alone group was the group without the added anti-SIGLEC 15 CAR T cells, and the NT group was added to non-transfected T cells. FIGS. 4 and 5 show results of cytokine release assays of anti-SIGLEC15 CAR cocultured with the substrate cells.

**Table 7**

| Target | Seq ID | ID |
|---|---|---|
| SIGLEC 15 | 49 | CAR6 |
| | 51 | CAR8 |
| | 52 | CAR9 |
| | 53 | CAR10 |
| | 46 | CAR5 |
| | 50 | CAR7 |
| | 54 | CAR11 |
| | 55 | CAR12 |
| | 56 | CAR13 |
| KIss1R | 71 | CAR14 |

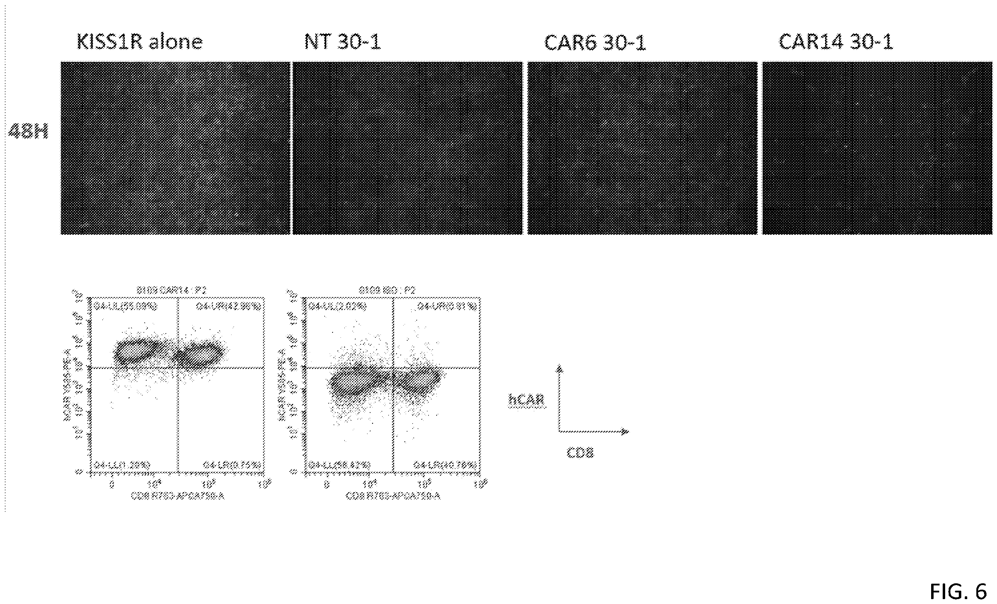

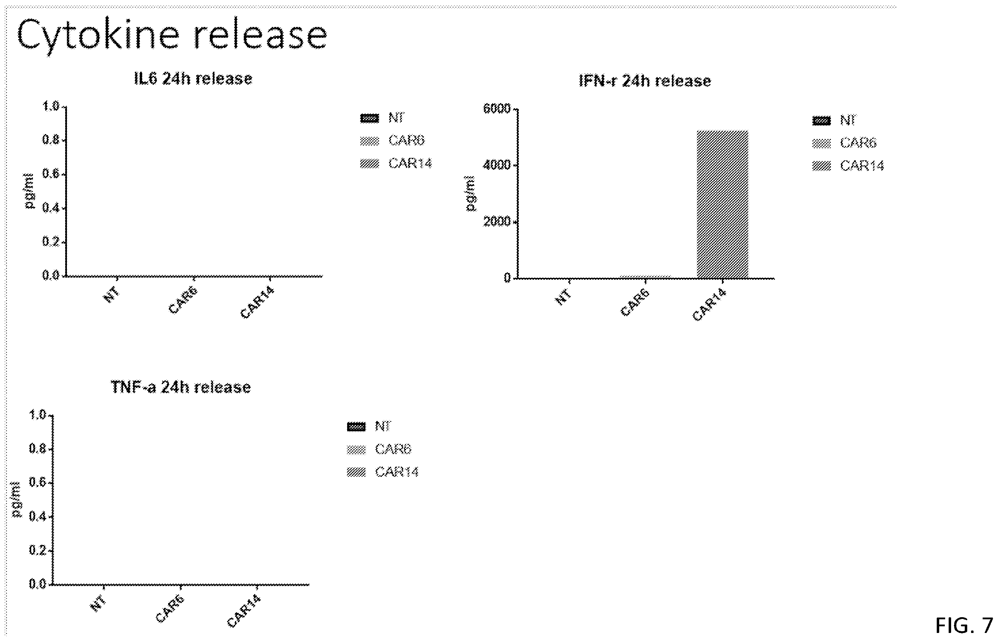

FIG. 6 shows results of a killing assay of anti-KISSR CAR (KISSR-CAR: SEQ ID NO: 71). It was observed that anti-KISS1R CAR T cells killed the substrate cells. The NT group was added to non-transfected T cells. FIG. 7 show results of cytokine release assays of anti-KISSR CAR T cells co-cultured with the substrate cells.

### ACPP Antibody Preparation and Anti-ACPP CAR

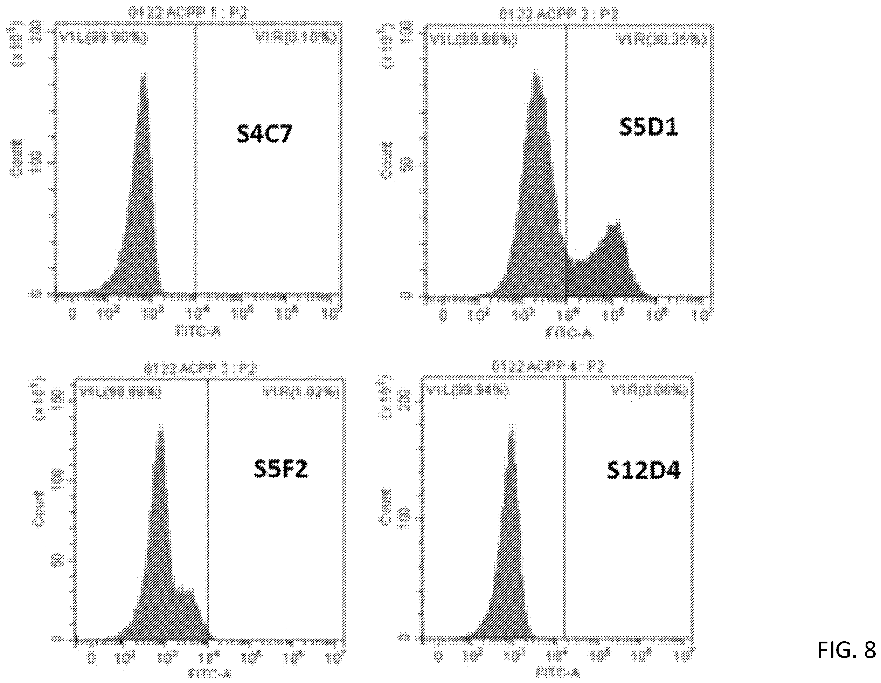

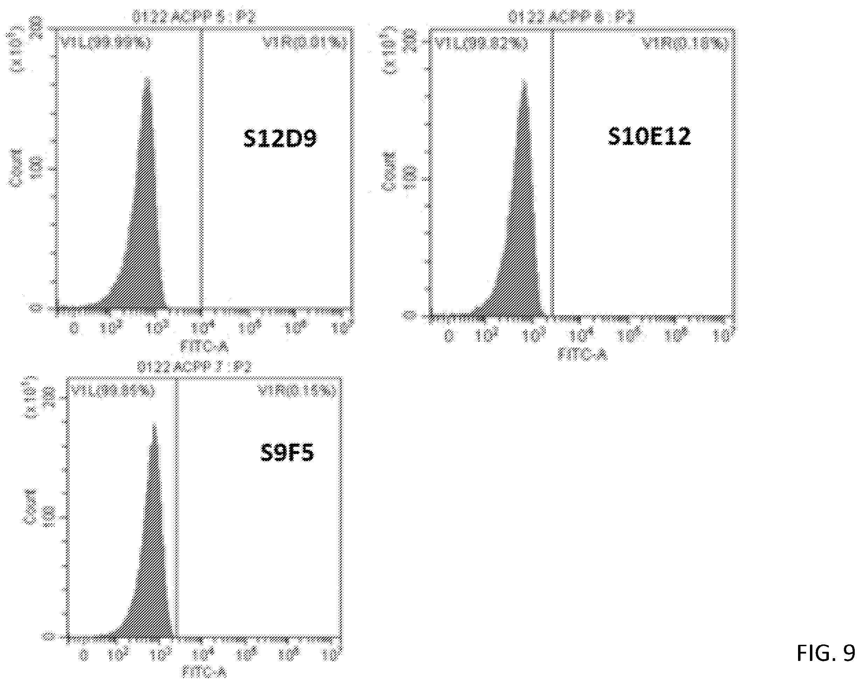

FIGS. 8-10 show flow cytometry assay for ACPP antibodies. 293T cells were transfected with the pcDNA-ACPP-flag plasmid to express the C-terminal flag-tagged ACPP protein. After 2 days, staining was detected using 7 ACPP antibodies and flag tag antibody staining. ACPP single staining does not break the membrane staining, first add ACPP primary antibody, then use goat anti human-FITC secondary antibody. When the flag label is single-dyed, APC direct-labeled primary antibody is used and the membrane is stained. Flag/ACPP double staining when ruptured. The Flag tag antibody detected 26.39% of the cells expressing the antigen. The S5D1 antibody detected 30.35% of cells expressing ACPP antigen. The double staining results showed that the positive cells had the same ACPP and flag expression levels. The staining results are specific. S5F2 also has a weaker binding, and the rest of the antibody flow has no signal. The transfected plasmid was able to express a C-terminal flag-tagged ACPP transmembrane protein on 293T cells. Flow cytometry experiments demonstrated that ACPP antibody S5D1 has good binding ability to antigen expressed on cell membrane. Flag labeling confirmed the normal expression of the protein.

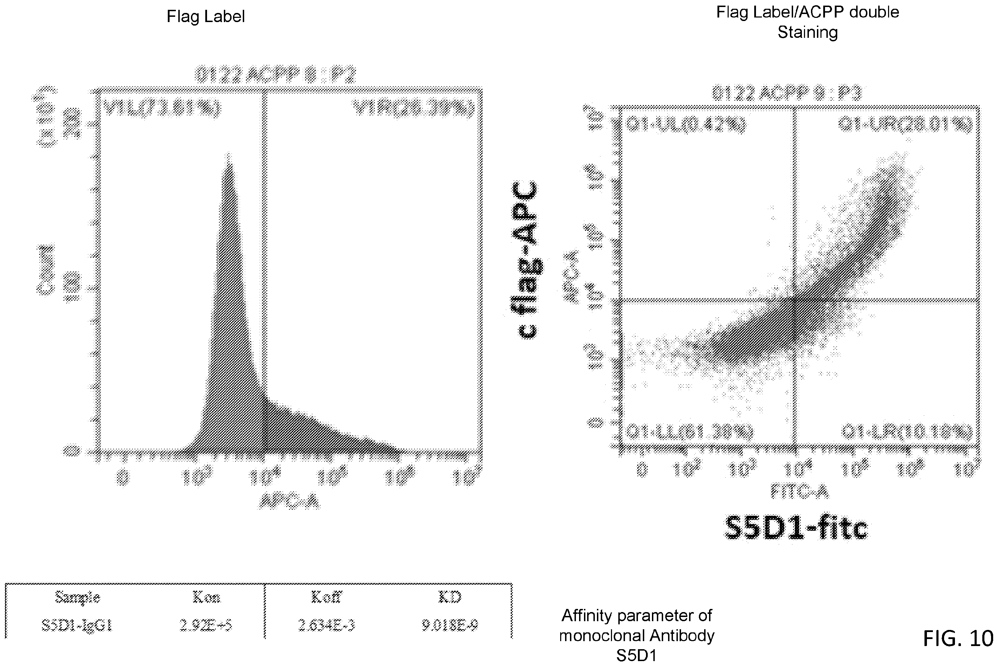

For the affinity assay in FIG. 10, the affinity of two monoclonal antibodies, S5D1 and S5F2, was analyzed using GE's BIAcore X-100 Biomolecular Interaction Analyzer. Affinity analysis was performed using a conventional procedure. The human monoclonal antibody S5D1 or S5F2 was captured using an anti-human antibody coated on a chip. Affinity analysis was then performed using different concentrations of recombinantly expressed PAP as the mobile phase. The affinity of the monoclonal antibody S5D1 is about 9 nM. It is speculated that the affinity of S5F2 is lower than 100 nM. S5D1 has a higher affinity for ACPP antigen. The sequences of the antibodies are listed in Table 6.

The ACPP antibodies were generated by cloning and expressing the selected serum prostatic acid phosphatase (PAP) antigen and screening using the prepared PAP antigen of the phage display human antibody library (humanphagedisplay). The ACPP gene sequence was cloned into the recombinant antigen expression vector PTSE-His. The constructed recombinant plasmid was transfected into HEK293 cells. The recombinant protein was purified using GE's Histrap FF affinity chromatography column. The human single-chain antibody library was screened using ACPP-His as an antigen with reference to a classical solid-phase screening strategy. The single-chain antibody library consists of a total of 12 sub-libraries of fully synthetic human single-chain antibody, natural human single-chain antibody and semi-synthetic-semi-natural single-chain antibody library. The total library capacity exceeds 10E9, and the correct rate is about 75%. Three rounds of routine screening were performed using solid phase coated antigens. After the second round of screening and after the third round of screening, 600 monoclonal clones (1200 monoclonal clones) were randomly selected for monoclonal identification. Approximately 40 positive monoclonal capable of specifically binding to PAP were obtained. Sequence analysis was performed on all monoclonal. The results showed that these positive clones shared 7 different antibody sequences. Representative clones are S4C7, S5D1, S5F2, S9F5, S10E12, S12D4 and S12D9, respectively (Sequences are listed in Table 6). The genes of the heavy and light chain variable region of the above seven scFvs were cloned into the eukaryotic expression vectors pTSEG1n and pTSEK, respectively. Seven human full antibodies were prepared using the HEK293 cell transient expression system. The recombinantly expressed whole antibody was purified using a Protein A affinity chromatography column.

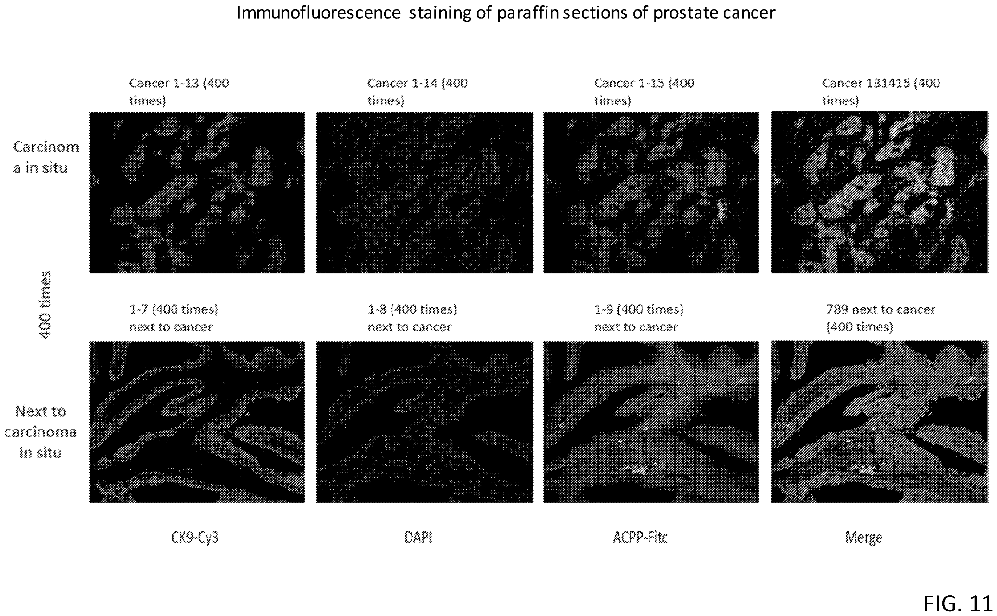

FIG. 11 shows immunofluorescence staining of paraffin sections of prostate cancer. The corresponding method includes: 1. tissue dehydration; 2. tissue transparency; 3. dipping wax; 4. embedding; 5. slicing and baking the slices; 6. sectioning and dewaxing; 7. antigen retrieval; 8. serum blocking; 9. adding primary antibody; 10. adding fluorescent secondary antibody; 11. serum blocking protecting from light; 12. adding primary antibody; 13. adding fluorescent secondary antibody; 14. complex dye core; and 15. collecting image. As shown, prostate cancer in situ and tissues adjacent to carcinoma in situ have higher ACPP expression. At the same time, the ACPP antibody used for the staining was S5D1, indicating that the antibody can specifically recognize ACPP. S5D1 was selected for further experiments below. The expression of ACPP in tumor tissue sections can be visualized by immunofluorescence staining, and it can also reflect the specificity and sensitivity of the antibody.

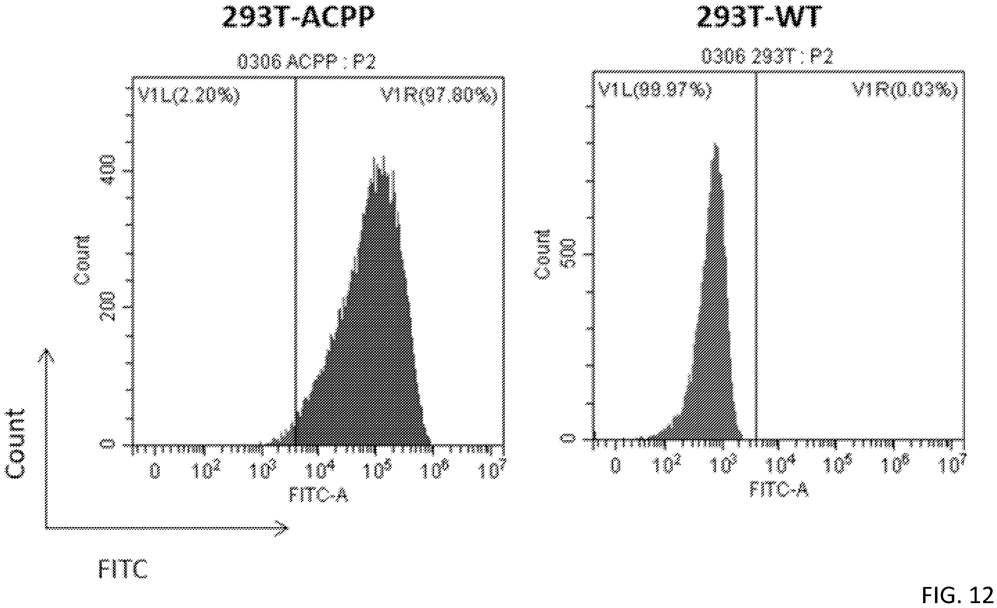

FIG. 12 shows 293T cells expressing ACPP. On day 0, the 293T-WT cells were digested and placed on a 6-well plate. On day 1, the cells were infected with the ACPP-lentivirus expression vector. On day 2, the medium was changed to remove the lentivirus, and fresh medium was added. The cells were digested and analyzed by flow cytometry. The antibody for flow cytometry was S5D1 and the antibody usage was 1 ug/100 ul. The flow results showed that the ACPP expression of 293T-ACPP cells was 97.80%. It can be used as an ACPP positive cell in subsequent experiments. ACPP can be overexpressed in 293T cells as an ACPP positive cell for experiments to assess the function of ACPP CAR T cells.

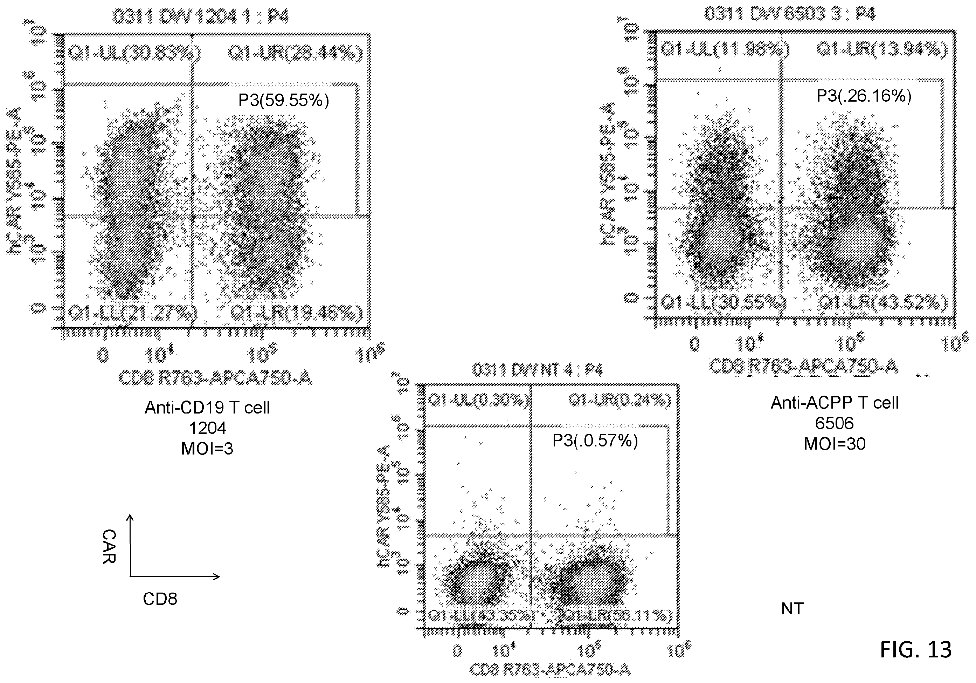

FIG. 13 shows flow cytometry assay of T cells expressing anti-ACPP CAR (anti-ACPP scFv: SEQ ID: 173). On day 0, peripheral blood from healthy volunteers was drawn; CD3+ T cells were sorted; and CD3/CD28 Dynabeads were added at a 1:1 ratio. On Day 1, T cells were transduced with vectors. CD19CAR T cells were obtained according to the infection ratio of MOI=13; ACPP CAR T cells were obtained according to the infection ratio of MOI=130. On day 2, culturing media were changed, and lentivirus was removed. The cells were then resuspended using fresh medium.

On day 6, flow cytometry was used to detect CAR expression. Since both CD19CAR and ACPP CAR include humanized antibodies, human CAR antibodies are used for detection. As a result, the expression of CD19 CAR in infected T cells was 59.55%, and the expression of ACPP CAR in infected T cells was 26.16%. Since both CD19CAR and ACPP CAR are humanized antibodies, the CAR expression was measured by using human CAR antibody for flow detection.

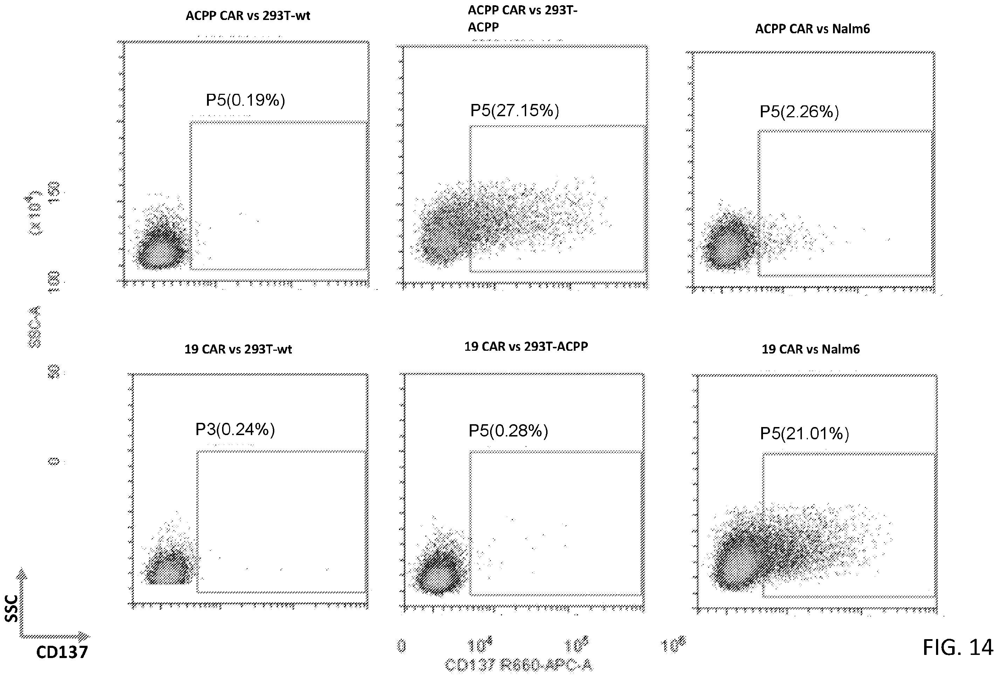

FIG. 14 shows results of co-culturing assay. The CAR ratio of ACPP CAR T cells and CD19CAR T cells was normalized with NT cells. 10⁴ CAR+ cells and 10⁴ 293T-WT or 293T-ACPP or Nalm-6 cells were co-cultured. CD137 expression of CAR positive CD8 positive cells was detected by flow cytometry after 48hrs. The left column is the CD137 expression of CAR T cells co-cultured with 293T-WT cells, and the CD137 expression is absent in both the CD19CAR group and the ACPP CAR group. This indicates that CAR T cells cannot be activated due to the absence of specific antigen expression in 293T. In the middle column, CAR T cells were co-cultured with 293T or 293T-ACPP cells overexpressing ACPP. The expression of CD137 in the ACPP CAR group was 27.15%, and that CD137 was not expressed in the CD19CAR group. This indicates that ACPP-CAR T Cells recognized and were activated by ACPP. The right column is a co-culture of CAR T cells with Nalm-6 cells, which are CD19-positive cells that are specifically recognized and activated by CD19CAR T cells. The results showed that the expression of CD137 in the CD19CAR group was 21.01%, and that CD137 was not expressed in the ACPP CAR group. The results are in line with expectations. Taken together, it was demonstrated that ACPP CAR T cells specifically recognize and can be activated by the ACPP antigen. CD137 is a marker protein for the activation of T cells, so the level of CD137 up-regulation of CAR T cells after co-culture with CAR T cells and substrate target cells can be used to determine whether CAR T cells are activated.

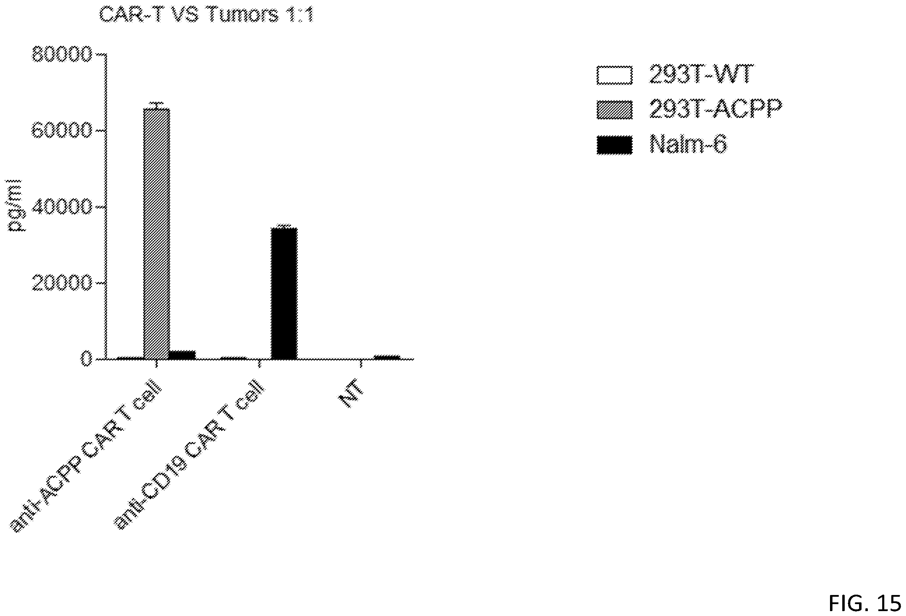

FIG. 15 shows IFN-γ release by ACPP CAR T cells in response to ACPP. The CAR ratio of ACPP CAR T cells and CD19 CAR T cells was normalized with NT cells. The experiment was carried out by co-culturing 0.2 or 10⁴ CAR+ cells and 10⁴ 293T-WT or 293T-ACPP or Nalm-6 cells. After 24hr, the supernatant was collected and the amount of IFN-γ released was examined. Nalm-6 is a CD19 positive cell, and 293T-ACPP is a 293T cell overexpressing ACPP. FIG 15 is a graph showing the results of CAR T cells: substrate cells of 1:1. ACPP CAR T cells exhibit significant IFN-γ release when co-cultured with 293T-ACPP, indicating that 293T-ACPP can be recognized by ACPP CAR T cells and release IFN-γ to kill target cells. At the same time, Nalm-6 can also be recognized by CD19CAR T cells and release IFN-γ to kill target cells. Furthermore, Nalm-6 did not stimulate the release of IFN-γ by ACPP CAR T cells. CD19 CAR T cells were also unable to stimulate IFN-γ release by 293T-ACPP. In sum, ACPP CAR T cells can specifically recognize ACPP-positive target cells and release IFN-γ to kill target cells under their stimulation. During the process of killing target cells by T cells, a large amount of cytokines such as IFN-γ are usually released to enhance the killing ability. Therefore, whether the CAR T cell has the ability to kill the target cell can be determined by the amount of IFN-γ released when the CAR T cell is co-cultured with the substrate target cell.

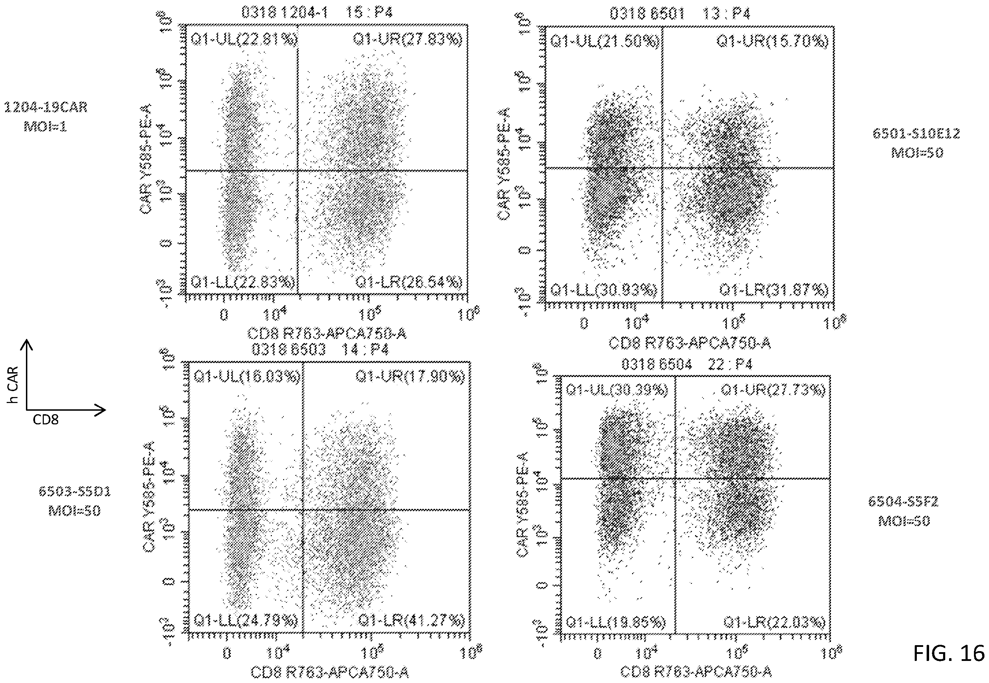

FIG. 16 shows that CAR expression of ACPP CAR on T cells can be measured by detecting human CAR antibody. On day 0, peripheral blood from healthy volunteers was drawn; CD3+ T cells were sorted; and CD3/CD28 Dynabeads were added in a 1:1 ratio. On Day 1, T cells were transducted with vectors. CD19CAR T cells were obtained according to the infection ratio of MOI=1; ACPP CAR T cells were obtained according to the infection ratio of MOI=50. On day 2, culturing media were changed and lentivirus was removed. The cells were then resuspended using fresh medium. On day 6, flow cytometry was used to detect CAR expression. Since both CD19CAR and ACPP CAR are humanized antibodies, human CAR antibodies are used for detection. The results are shown in the figure. The expression of CD19CAR in CD19CAR T cells was 50.64%, and the expression of ACPP in ACPP CAR T cells (6501) was 37.2%, in ACPP CAR T cells (6503) was 33.93%, and in ACPP CAR T cells (6504) was 45.63%. Since both 19CAR and ACPP CAR are humanized antibodies, the CAR expression level can be obtained by using human CAR antibody for flow detection.

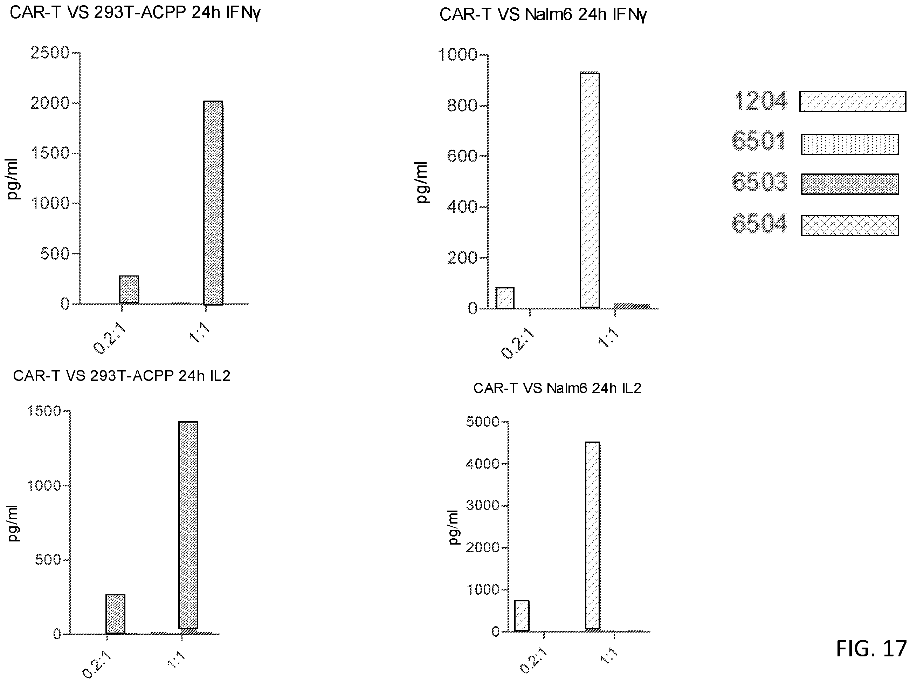

FIG. 17 shows cytokine release by ACPP CAR T cells in response to ACPP. The CAR ratio of ACPP CAR T cells and CD19 CAR T cells was normalized with NT cells. The experiment was carried out with 0.2 or 1× 10⁴CAR+ cells co-cultured with 1 × 10⁴ 293T-ACPP or Nalm-6 cells, and the supernatant was collected after 24hrs to detect IFN-γ and IL2. Nalm-6 is a CD19 positive cell, and 293T-ACPP is a 293T cell overexpressing ACPP. FIG 17 shows that ACPP CAR T 6503 exhibits significant increased release of IFN-γ and IL2 when co-cultured with 293T-ACPP, indicating that ACPP CAR T 6503 cells recognize 293T-ACPP and activate the release of IFN-γ and IL2. In contrast, ACPP CAR T 6501 and 6504 cells could not activate the release of IFN-γ and IL2. At the same time, CD19CAR T cells were able to recognize Nalm-6 and activate the release of IFN-γ and IL2. Furthermore, Nalm-6 failed to activate ACPP CAR T cells to release IFN-γ. At the same time, CD19CAR T cells could not be activated by 293T-ACPP to release IFN-γ. This indicates that the two targeted CAR T cells are specific. During the process of killing the target cells by T cells, a large amount of cytokines such as IFN-γand IL2 are usually released to enhance the killing ability. Therefore, whether the CAR T cells have a killing ability against the target cells can be determined by the release amount of IFN-γ and IL2 when the CAR T cells are co-cultured with the substrate target cells.

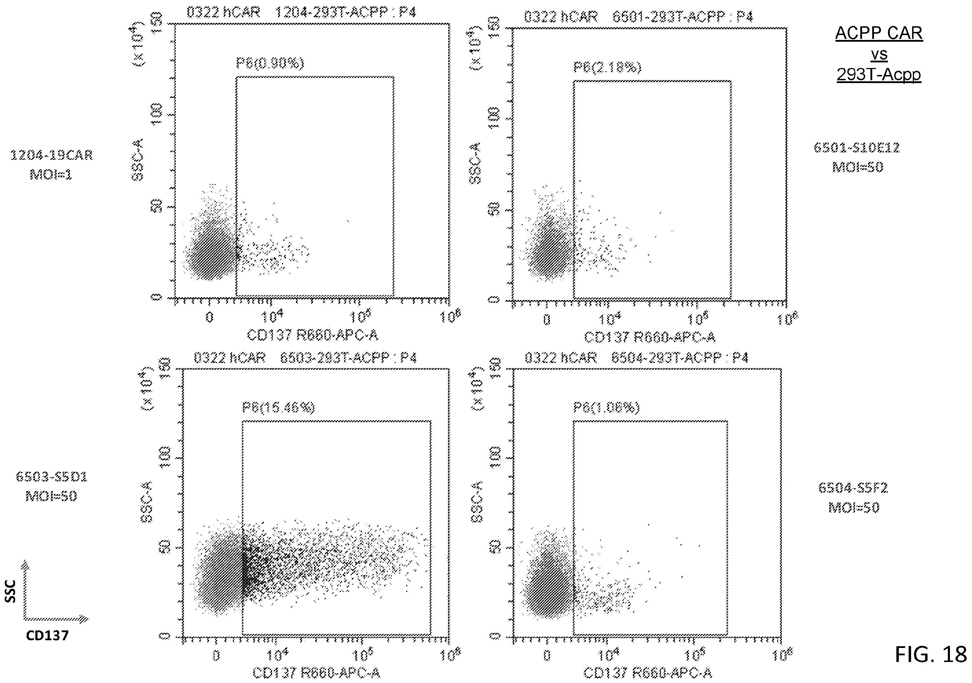

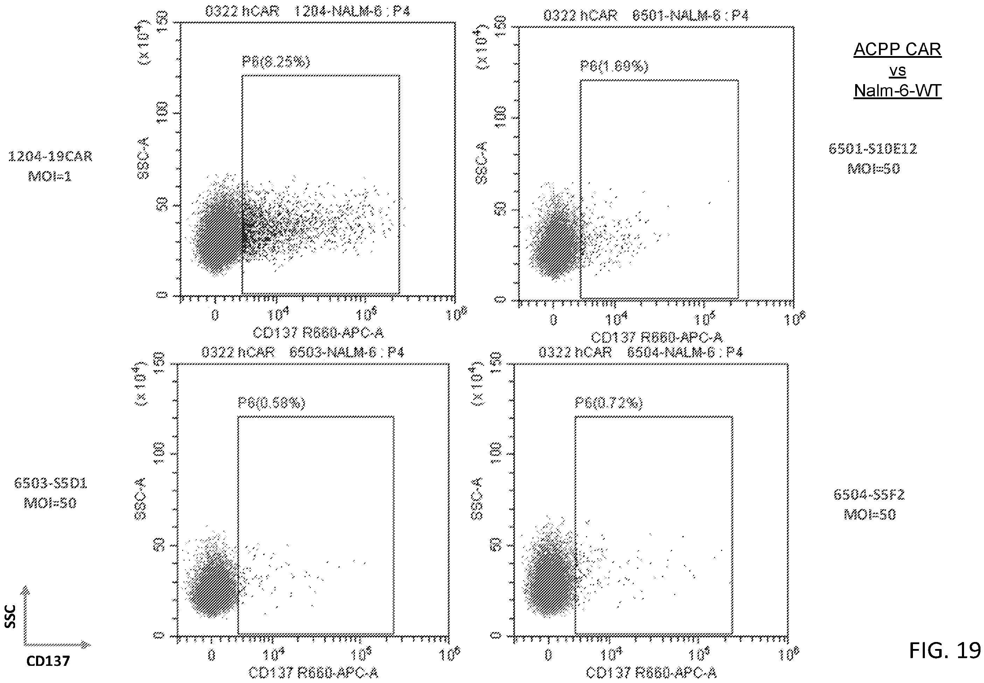

FIGS. 18 and 19 show CD137 expression in various conditions. CAR 1204 is a human-derived CD19CAR, which can be labeled with human CAR antibody and CD137 antibody. The CAR ratio of ACPP CAR T cells and CD19CAR T cells was normalized with NT cells. 10⁴ CAR+ cells were co-cultured with 10⁴ 293T-ACPP or Nalm-6 cells, and CD137 expression of CD8 positive cells was detected by flow cytometry after 24hrs. The first row is the result of co-culture of CAR T cells and 293T-ACPP. CD19CAR T cells were not activated by 293T-ACPP to express CD137. ACPP CAR T 6503 cells were activated by 293T-ACPP to express CD137. ACPP CART 6501 and 6504 cells were not activated by 293T-ACPP to express CD137. The second row is the result of co-culture of CAR T cells and the CD19 positive cell line Nalm6. CD19CAR T cells can be activated by Nalm6 to express CD137. All ACPP CAR T cells could not be activated by Nalm6 to express CD137, indicating that CD19CAR T and ACPP CAR T cells are specific. CD137 is a marker protein for the activation of T cells. Thus, it is determined that CAR T cells are activated by detecting the expression of CD137 in CAR T cells after co-culture of CAR T cells and substrate target cells.

### UPK2 Antibody Preparation

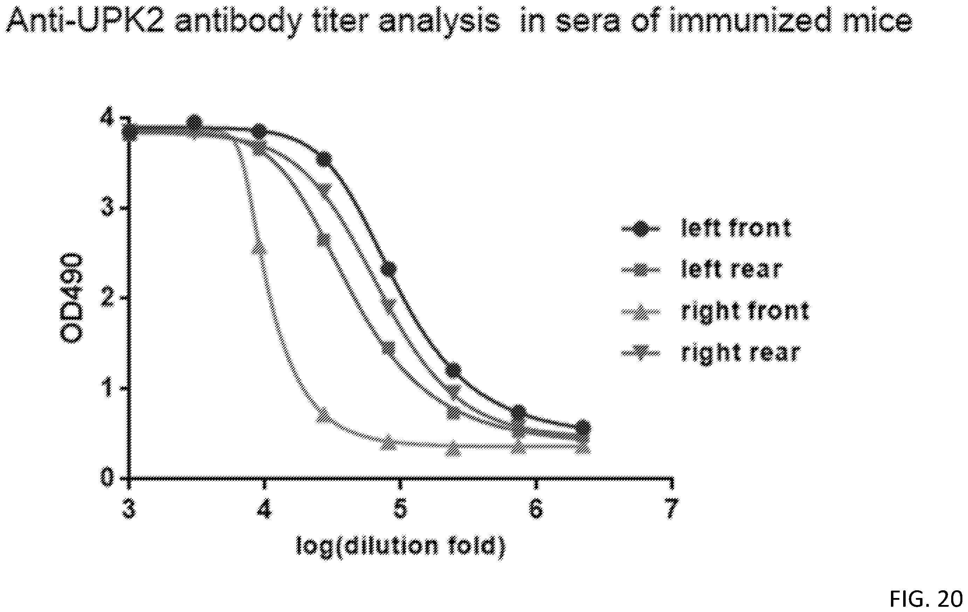

The recombinant UPK2 extracellular domain (UPK2-His) was prepared using E. coli expression system. BALB/c mice of 6-8 weeks old were taken, and the mice were subjected to tail vein blood sampling to leave the background serum before immunization. The UPK2-His recombinant antigen was immunized for the first time and emulsified with complete Freund's adjuvant. Each mouse was intraperitoneally injected with 50 µg of recombinant antigen. The immunization was boosted at intervals of two weeks, and UPK2-His recombinant antigen was emulsified by incomplete adjuvant. Each mouse was intraperitoneally injected with 50 µg of recombinant antigen for three booster immunizations. The tail vein blood collection was performed before the third booster immunization to analyze the serum UPK2 antibody titer. The results showed that the antibody titer of UPK2 in the serum of three of the four mice reached 10⁶ or more (Fig. 20), indicating that the immunization was successful.

The fifth immunization was changed to shock the immunization, and the UPK2-His recombinant antigen without adjuvant was used as the immunogen. Each mouse was intraperitoneally injected with 50 µg of recombinant antigen, and the mice were sacrificed 3 days after the immunization. Spleen cells were collected from mice and labeled as "left front" in the graph shown in FIG. 20.

The mouse spleen lymphocytes were separated using mouse lymphocyte separation solution (Dakko, CAT#DKW33-R0100), and the isolated lymphocytes were totaled using the total RNA extraction kit (Tiangen, CAT#DP430). RNA extraction. Using the extracted total RNA as a template, the first strand cDNA synthesis kit (Thermo scientific, CAT#K1621) was used to synthesize the heavy chain variable region and the light chain variable region, respectively. The reverse transcription primers were gene-specific primers, and the primer pairing was performed. The regions are located in the antibody heavy chain constant region and the antibody light chain constant region, respectively, and the specific sequences are PmCGR: TGCATTTGAACTCCTTGCC (SEQ ID NO: 189) and PmCKR: CCATCAATCTTCCACTTGAC (SEQ ID NO: 190), respectively. The synthesized cDNA was immediately stored at -70 °C for storage. Then, the cDNA was obtained by reverse transcription and used as a template to obtain the primers (Journal of Immunological Methods, 201 (1997), 35-55), and the murine antibodies VH and VK were amplified by PCR, respectively. The overlap extension PCR technique was used to construct a single chain antibody (scFv). Finally, the prepared mouse single-chain antibody gene was cloned into the vector pADSCFV-S to construct a ScFv library. The library capacity of this antibody library reached 1.6×10⁸, and the correct rate was 41.5%.

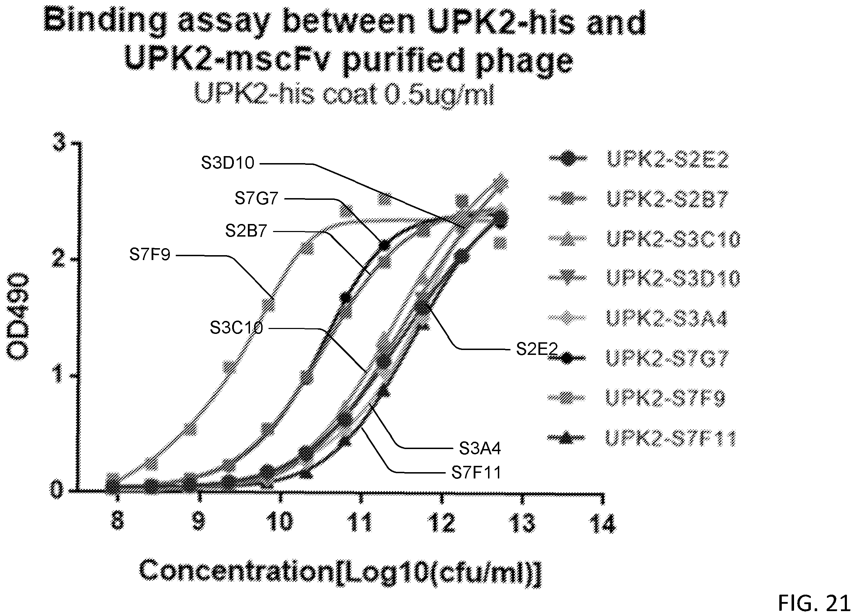

Using recombinant UPK2-his as the antigen, the mouse single-chain antibody library was screened by reference to the classical solid-phase screening strategy, and three rounds of screening were performed by means of binding, elution, neutralization, infection and amplification, in the second round. After the third round of screening, about 700 monoclonal clones were identified by phage ELISA. Sixty clones with high positive ELISA signal were selected for sequence analysis to obtain eight strains with different sequences that bind to UPK2-His. The eight antibodies are: clone S2B7, S2E2, S3A4, S3C10, S3D10, S7F9, S7F11 and S7G7 (FIG. 21). The heavy and light chain variable region sequences (scFv) of the eight monoclonal antibodies are shown in Table 6.

The heavy and light chain variable region genes of the above eight scFvs were cloned into the eukaryotic expression vectors pTSEG1n and pTSEK, respectively, and eight murine-human chimeric antibodies (murine antibody variable regions) were prepared using the company's HEK293 cell transient expression system. Human antibody constant region). The recombinant whole antibody was purified by Protein A affinity chromatography column, and SDS-PAGE showed. These eight antibodies were normally expressed, and their purity met the level for protein identification.

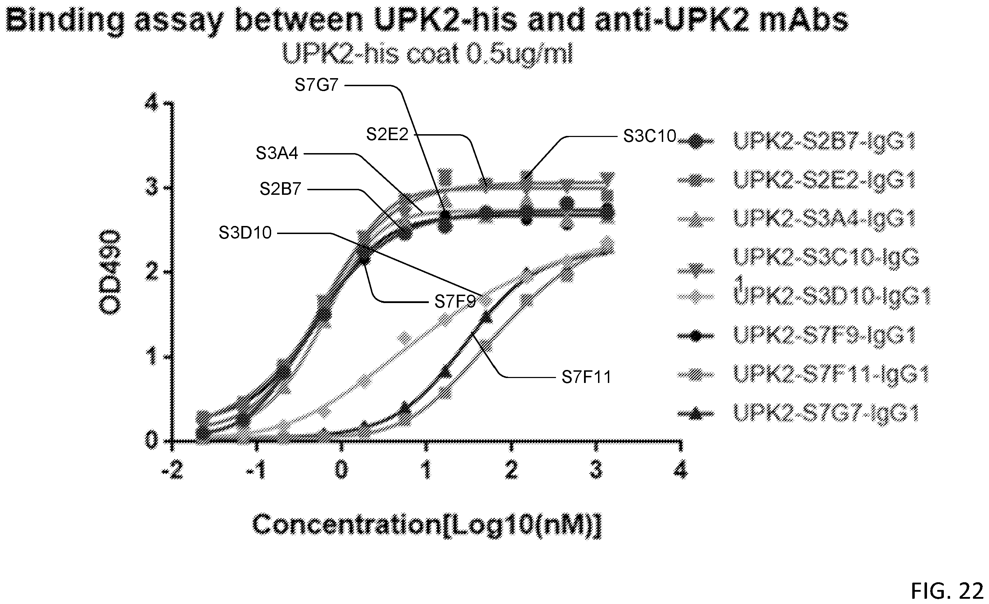

The ability of recombinant whole antibodies to bind antigen UPK2-His was analyzed using a classical ELISA method. The results are shown in FIG. 22. As shown in FIG. 22, all 8 antibodies can bind to the antigen UPK2-His.

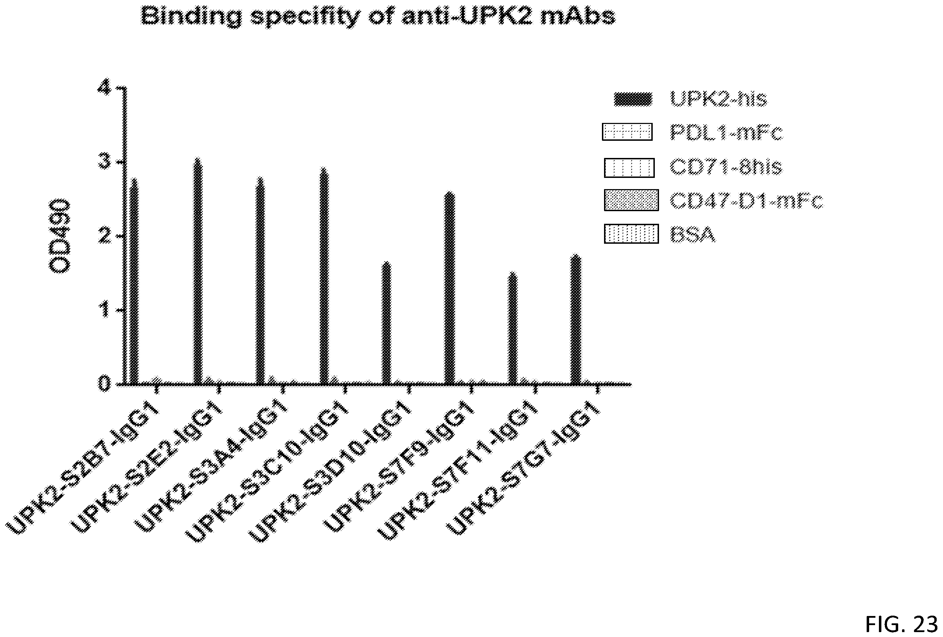

At the same time, the specificity of the prepared monoclonal antibody was analyzed by a similar ELISA method. As shown in FIG. 23, the eight recombinant antibodies can specifically recognize the recombinant UPK2-His, and there are no obvious non-specific bindings of various unrelated antigens.

The affinity of the eight monoclonal antibodies was analyzed using GE's BIAcore X-100. Affinity analysis was performed using a conventional procedure by first capturing the human monoclonal antibody with an anti-human antibody coated on a chip, and then using different concentrations of recombinant UPK2-His as the mobile phase for affinity analysis. As shown in Table 8, the affinity (KD) of these recombinant antibodies is mostly between 0.1 nM and 10 nM. Among them, the binding and dissociation of S7F11/S7G7 were slow, and the binding of S3D10 was too slow. When the three monoclonal antibodies were analyzed by BIAcore X-100 for affinity analysis, the automatic fitting of KD was poor, and the data was for reference only.

**Table 8: Recombinant anti-UPK2 monoclonal antibody affinity parameters determined by BIAcore**

| Monoclonal antibody | Kon | Koff | KD | Remarks |
|---|---|---|---|---|
| S2B7 | 1.493×10⁵ | 3.495×10⁻³ | 2.34×10⁸ | |
| S3A4 | 1.125 ×10⁵ | 3.05 ×10⁻³ | 2.712×10⁻⁸ | |
| S7F9 | 1.4 ×10⁵ | 2.344 ×10⁻³ | 1.674 ×10⁻⁸ | |
| S2E2 | 1.381 ×10⁶ | 3.894 ×10⁻³ | 2.82 ×10⁻⁹ | |
| S3C10 | 1.226 ×10⁶ | 1.117 ×10⁻³ | 9.109 ×10⁻¹⁰ | |
| S7F11 | 3.504 ×10³ | 8.126 ×10⁻⁶ | 2.319 ×10⁻⁹ | Kd overrun |
| S7G7 | 5.049 ×10⁴ | 1.202 ×10⁻⁶ | 2.381 ×10⁻¹¹ | Kd overrun |
| S3D10 | 6.431 ×10² | 2.614 ×10⁻³ | 4.064 ×10⁻⁶ | U-value=15 |

In this study, the recombinant UPK2 extracellular domain (UPK2-His) was used to complete the immunization of mice, the construction, screening and monoclonal identification of the murine immune library. Eight murine monoclonal antibodies (S2B7, S2E2, S3A4, S3C10, S3D10, S7F9, S7F11 and S7G7), with different sequences and capable of binding recombinant UPK2 were obtained, and preliminary specificity analysis showed that these monoclonal antibodies specifically bind to recombinant UPK2-his. Affinity analysis based on BIAcore showed that the affinity of these recombinant anti-UPK2 antibodies was mostly between 0.1 nM and 10 nM.

## Claims

1. An antibody that binds prostatic acid phosphatase (ACPP), wherein the antibody comprises:
a heavy chain variable region (HVR) comprising amino acid sequence SEQ ID NO: 83 and a light chain variable region (LVR) comprising amino acid sequence SEQ ID NO: 82.

2. The antibody of claim 1, wherein the HVR is joined to a human IgG chain constant region, and optionally the human IgG is IgG1 or IgG3.

3. The antibody of claim 1, wherein the antibody is conjugated to a cytotoxic agent, and optionally the cytotoxic agent is a radioactive isotope or a toxin.

4. The antibody of claim 1, wherein the antibody is a scFv, and the LVR is connected to HVR via a linker.

5. A chimeric antigen receptor (CAR) comprising an antigen binding domain comprising the antibody of one of claims 1 or 4.

6. The chimeric antigen receptor (CAR) of claim 5, wherein the scFv comprises the amino acid sequence SEQ ID NO: 173.

7. The CAR of claim 5, wherein the CAR comprises an extracellular domain, a transmembrane domain, and an intracellular domain, the extracellular domain comprising the antigen binding domain.

8. The CAR of claim 7, wherein the intracellular domain comprises a co-stimulatory signaling region that comprises an intracellular domain of a co-stimulatory molecule selected from the group consisting of CD27, CD28, 4-1BB, OX40, CD30, CD40, PD-1, ICOS, lymphocyte function-associated antigen-1 (LFA-1), CD2, CD7, LIGHT, NKG2C, B7-H3, and a combination thereof.

9. A polynucleotide that encodes the antibody of any one of claims 1-4.

10. A polynucleotide that encodes the CAR of any one of claims 5-8.

11. A modified cell comprising the polynucleotide of claim 9 or 10.

## Patentansprüche

1. Ein Antikörper, der Prostatasäurephosphatase (ACPP) bindet, wobei der Antikörper Folgendes umfasst:
eine variable Region der schweren Kette (HVR), umfassend die Aminosäuresequenz SEQ ID NO: 83, und eine variable Region der leichten Kette (LVR), umfassend die Aminosäuresequenz SEQ ID NO: 82.

2. Der Antikörper nach Anspruch 1, wobei die HVR mit einer konstanten Region einer menschlichen IgG-Kette verbunden ist und das menschliche IgG optional IgG1 oder IgG3 ist.

3. Der Antikörper nach Anspruch 1, wobei der Antikörper an ein cytotoxisches Mittel konjugiert ist und das cytotoxische Mittel gegebenenfalls ein radioaktives Isotop oder ein Toxin ist.

4. Der Antikörper nach Anspruch 1, wobei der Antikörper ein scFv ist und die LVR über einen Linker mit der HVR verbunden ist.

5. Ein chimärer Antigen-Rezeptor (CAR), welcher eine Antigenbindungsdomäne umfasst , die den Antikörper nach einem der Ansprüche 1 oder 4 umfasst.

6. Der chimäre Antigenrezeptor (CAR) nach Anspruch 5, wobei das scFv die Aminosäuresequenz SEQ ID NO: 173 umfasst.

7. Der CAR nach Anspruch 5, wobei der CAR eine extrazelluläre Domäne, eine Transmembrandomäne und eine intrazelluläre Domäne umfasst, wobei die extrazelluläre Domäne die Antigenbindungsdomäne umfasst.

8. Der CAR nach Anspruch 7, wobei die intrazelluläre Domäne eine kostimulatorische Signalregion umfasst, die eine intrazelluläre Domäne eines kostimulatorischen Moleküls umfasst, das aus der Gruppe bestehend aus CD27, CD28, 4-1BB, OX40 , CD30, CD40, PD-1, ICOS, Lymphozyten-Funktions-assoziiertes Antigen-1 (LFA-1), CD2, CD7, LIGHT, NKG2C, B7-H3 und einer Kombination davon besteht.

9. Ein Polynukleotid, das den Antikörper gemäß einem der Ansprüche 1 bis 4 codiert.

10. Ein Polynukleotid, das den CAR gemäß einem der Ansprüche 5 bis 8 codiert.

11. Eine modifizierte Zelle, die das Polynukleotid gemäß Anspruch 9 oder 10 umfasst.

## Revendications

1. Anticorps qui se lie à la phosphatase acide prostatique (ACPP), dans lequel l'anticorps comprend :
une région variable de chaîne lourde (HVR) comprenant la séquence d'acides aminés SEQ ID NO : 83 et une région variable de chaîne légère (LVR) comprenant la séquence d'acides aminés SEQ ID NO : 82.

2. Anticorps selon la revendication 1, dans lequel la HVR est reliée à une région constante de chaîne IgG humaine, et facultativement l'IgG humaine est IgG1 ou IgG3.

3. Anticorps selon la revendication 1, dans lequel l'anticorps est conjugué à un agent cytotoxique, et facultativement l'agent cytotoxique est un isotope radioactif ou une toxine.

4. Anticorps selon la revendication 1, dans lequel l'anticorps est un scFv, et la LVR est connectée à la HVR via un lieur.

5. Récepteur antigénique chimérique (CAR) comprenant un domaine de liaison à l'antigène comprenant l'anticorps selon l'une des revendications 1 ou 4.

6. Récepteur antigénique chimérique (CAR) selon la revendication 5, dans lequel le scFv comprend la séquence d'acides aminés SEQ ID NO : 173.

7. CAR selon la revendication 5, dans lequel le CAR comprend un domaine extracellulaire, un domaine transmembranaire, et un domaine intracellulaire, le domaine extracellulaire comprenant le domaine de liaison à l'antigène.

8. CAR selon la revendication 7, dans lequel le domaine intracellulaire comprend une région de signalisation co-stimulatrice qui comprend un domaine intracellulaire d'une molécule co-stimulatrice choisie parmi le groupe constitué de CD27, CD28, 4-1BB, OX40, CD30, CD40, PD-1, ICOS, antigène-1 associé à la fonction lymphocytaire (LFA-1), CD2, CD7, LIGHT, NKG2C, B7-H3, et d'une combinaison de ceux-ci.

9. Polynucléotide qui code pour l'anticorps selon l'une quelconque des revendications 1 à 4.

10. Polynucléotide qui code pour le CAR selon l'une quelconque des revendications 5 à 8.

11. Cellule modifiée comprenant le polynucléotide selon la revendication 9 ou 10.
